(19) Europäisches Patentamt
European Patent Office
Office européen des brevets

(11) EP 1 508 327 B1

# (12) EUROPÄISCHE PATENTSCHRIFT

(45) Veröffentlichungstag und Bekanntmachung des Hinweises auf die Patenterteilung:
**07.05.2014 Patentblatt 2014/19**

(21) Anmeldenummer: **04015739.8**

(22) Anmeldetag: **05.07.2004**

(51) Int Cl.:
*A61K 31/35* (2006.01) *A23L 1/30* (2006.01)
*A61K 8/49* (2006.01) *A61Q 5/00* (2006.01)
*A61Q 19/00* (2006.01) *A61Q 19/02* (2006.01)
*A61Q 19/08* (2006.01) *A61K 45/06* (2006.01)
*A61K 31/352* (2006.01) *A61Q 11/00* (2006.01)
*A61Q 17/04* (2006.01)

(54) **Verwendung von 5,7-Dihydroxy-2-methylchromen-4-on zur Hautpflege**

Use of 5,7-dihydroxy-2-methylchromen-4-on for the care of the skin

Utilisation de 5,7-dihydroxy-2-méthylchromen-4-one pour le soin de la peau

(84) Benannte Vertragsstaaten:
**AT BE BG CH CY CZ DE DK EE ES FI FR GB GR HU IE IT LI LU MC NL PL PT RO SE SI SK TR**

(30) Priorität: **18.08.2003 DE 10337863**

(43) Veröffentlichungstag der Anmeldung:
**23.02.2005 Patentblatt 2005/08**

(73) Patentinhaber: **Merck Patent GmbH**
**64293 Darmstadt (DE)**

(72) Erfinder:
- **Carola, Christophe, Dr.**
  **63225 Langen (DE)**
- **Huber, Sylvia**
  **64291 Darmstadt (DE)**
- **Rosskopf, Ralf**
  **64839 Münster (DE)**
- **Buchholz, Herwig, Dr.**
  **60599 Frankfurt (DE)**

(56) Entgegenhaltungen:
**WO-A-00/26206 WO-A-02/066001**
**DE-A- 2 132 260 DE-A1- 2 132 260**

- **DATABASE HCAPLUS ACS; 2. April 1994 (1994-04-02), XP002308587 gefunden im STN Database accession no. 120:173133/DN & JP 05 301813 A (KAO CORP.) 16. November 1993 (1993-11-16)**
- **DATABASE HCAPLUS ACS; 4. August 1997 (1997-08-04), XP002308588 gefunden im STN Database accession no. 127:140192/DN & JP 09 188608 A (KAO CORP.) 22. Juli 1997 (1997-07-22)**
- **DATABASE HCAPLUS ACS; 14. Mai 1998 (1998-05-14), XP002308589 gefunden im STN Database accession no. 129:32141/DN & JP 10 114640 A (KAO CORP.) 6. Mai 1998 (1998-05-06)**
- **DATABASE HCAPLUS ACS; 15. Oktober 1997 (1997-10-15), XP002308590 gefunden im STN Database accession no. 127:298458/DN -& DATABASE WPI Section Ch, Week 199749 Derwent Publications Ltd., London, GB; Class B05, AN 1997-535421 XP002308794 -& WO 97/35618 A1 (KAO CORP) 2. Oktober 1997 (1997-10-02)**
- **OONUMA HIROAKI ET AL: "TRANSDERMAL PREPARATIONS CONTAINING CHROMONE DERIVATIVES OR THEIR SALTS MELANIN-FORMATION INHIBITORS", HCAPLUS, XP002308587, & JP 5 301813 A (KAO CORP.) 16 November 1993 (1993-11-16)**
- **PATENT ABSTRACTS OF JAPAN 1998 & JP 1998, 10114640, A, (KAO CORP.), 6 May 1998 (1998-05-06)**
- **OONUMA HIROAKI ET AL: "TRANSDERMAL PREPARATIONS CONTAINING CHROMONE DERIVATIVES OR THEIR SALTS MELANIN-FORMATION INHIBITORS", HCAPLUS, XP002308587, & JP 5 301813 A (KAO CORP.) 16 November 1993 (1993-11-16)**
- **OKAMOTO YOSHIMASA ET AL: "HAIR PREPARATIONS CONTAINING CHROMONES FOR INHIBITION OF GRAY HAIR", HCAPLUS, XP002308588, & JP 9 188608 A (KAO CORP.) 22 July 1997 (1997-07-22)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- **OONUMA HIROAKI ET AL: "TRANSDERMAL PREPARATIONS CONTAINING CHROMONE DERIVATIVES OR THEIR SALTS MELANIN-FORMATION INHIBITORS", HCAPLUS, XP002308587, & JP 5 301813 A (KAO CORP.) 16 November 1993 (1993-11-16)**
- **OKAMOTO YOSHIMASA ET AL: "HAIR PREPARATIONS CONTAINING CHROMONES FOR INHIBITION OF GRAY HAIR", HCAPLUS, XP002308588, & JP 9 188608 A (KAO CORP.) 22 July 1997 (1997-07-22)**
- **KAWAKAMI KYOKO: "MAKE-UP STOCK CONTAINING INCOMPATIBLE OILS WITH PROLONGED STORAGE STABILITY", HCAPLUS, XP002308589, & JP 10 114640 A (KAO CORP.) 6 May 1998 (1998-05-06)**
- **MURASE TAKATOSHI ET AL: "DERMATOLOGIC PREPARATION", HCAPLUS, XP002308590, & DATABASE WPI Section Ch, Week 199749 Thomson Scientific, London, GB; Class B05, AN 1997-535421 XP002308794, & WO 97/35618 A1 (KAO CORP) 2 October 1997 (1997-10-02)**
- **DATABASE CAPLUS [Online] CHEMICAL ABSTRACTS SERVICE, COLUMBUS, OHIO, US; 29 November 2002 (2002-11-29), XP002309296, retrieved from STN Database accession no. 2002: 906296**
- **OONUMA HIROAKI ET AL: "TRANSDERMAL PREPARATIONS CONTAINING CHROMONE DERIVATIVES OR THEIR SALTS MELANIN-FORMATION INHIBITORS", HCAPLUS, XP002308587, & JP 5 301813 A (KAO CORP.) 16 November 1993 (1993-11-16)**
- **OKAMOTO YOSHIMASA ET AL: "HAIR PREPARATIONS CONTAINING CHROMONES FOR INHIBITION OF GRAY HAIR", HCAPLUS, XP002308588, & JP 9 188608 A (KAO CORP.) 22 July 1997 (1997-07-22)**
- **OONUMA HIROAKI ET AL: "TRANSDERMAL PREPARATIONS CONTAINING CHROMONE DERIVATIVES OR THEIR SALTS MELANIN-FORMATION INHIBITORS", HCAPLUS, XP002308587, & JP 5 301813 A (KAO CORP.) 16 November 1993 (1993-11-16)**
- **OONUMA HIROAKI ET AL: "TRANSDERMAL PREPARATIONS CONTAINING CHROMONE DERIVATIVES OR THEIR SALTS MELANIN-FORMATION INHIBITORS", HCAPLUS, XP002308587, & JP 5 301813 A (KAO CORP.) 16 November 1993 (1993-11-16)**
- **OONUMA HIROAKI ET AL: "TRANSDERMAL PREPARATIONS CONTAINING CHROMONE DERIVATIVES OR THEIR SALTS MELANIN-FORMATION INHIBITORS", HCAPLUS, XP002308587, & JP 5 301813 A (KAO CORP.) 16 November 1993 (1993-11-16)**


Bemerkungen:

Die Akte enthält technische Angaben, die nach dem Eingang der Anmeldung eingereicht wurden und die nicht in dieser Patentschrift enthalten sind.

## Beschreibung

Die vorliegende Erfindung betrifft die nicht-therapeutische Verwendung von Chromen-4-on-Derivaten zur Pflege, Konservierung oder Verbesserung des allgemeinen Zustandes der Haut oder Haare und zur Prophylaxe gegen zeit-und/oder lichtinduzierte Alterungsprozesse der menschlichen Haut oder menschlicher Haare und zur Prophylaxe und/oder Behandlung von Hautkrankheiten. Ferner betrifft die Erfindung Zubereitungen mit einem wirksamen Gehalt an Chromen-4-on-Derivaten. Insbesondere betrifft die vorliegende Erfindung kosmetische Zubereitungen zur Prophylaxe gegen Alterungsprozesse in der Haut.

Die menschliche Haut unterliegt gewissen Alterungsprozessen, die teilweise auf intrinsische Prozesse (chronoaging) und teilweise auf exogene Faktoren (environmental, z.B. photoaging) zurückzuführen sind. Zusätzlich können vorübergehende oder auch andauernde Veränderungen des Hautbildes auftreten, wie Akne, fettige oder trockene Haut, Keratosen, Rosaceae, lichtempfindliche, entzündliche, erythematöse, allergische oder autoimmunreaktive Reaktionen wie Dermatosen und Photodermatosen.

Zu den exogenen Faktoren zählen insbesondere das Sonnenlicht oder künstliche Strahlungsquellen mit vergleichbarem Spektrum sowie Verbindungen, die durch die Strahlung entsteher können, wie undefinierte reaktive Photoprodukte, die auch radikalisch oder ionisch sein können. Zu diesen Faktoren zählen auch Zigarettenrauch und die darin enthaltenen reaktiven Verbindungen wie Ozon, freie Radikale, beispielsweise das Hydroxylradikal, Singutettsauerstoff und andere reaktive Sauerstoff- oder Stickstoffverbindungen, die die natürliche Physiologie oder Morphologie der Haut stören.

Durch den Einfluß dieser Faktoren kann es unter anderem zu direkten Schäden an der DNA der Hautzellen kommen sowie an den Kollagen-, Elastin- oder Glycosaminoglycanmolekülen der extrazellulären Matrix, die für die Festigkeit der Haut verantwortlich sind. Darüberhinaus kann es zu einer Beeinflussung der Signaltransduktionsketten kommen, an deren Ende die Aktivierung matrixabbauender Enzyme steht. Wichtige Vertreter dieser Enzyme sind die Matrixmetalloproteinasen (MMPs, z.B. Kollagenasen, Gelatinasen, Stromelysine), deren Aktivität zusätzlich durch TIMPs (tissue inhibitor of matrix metalloproteinases) reguliert wird.

Die Folgen der o.g. Alterungsprozesse sind Verdünnung der Haut, schwächere Verzahnung von Epidermis und Dermis, Reduktion der Zellzahl sowie der versorgenden Blutgefäße. Dabei kommt es zur Ausbildung von feinen Linien und Falten, die Haut wird ledrig und es können Pigmentstörungen auftreten.

Die gleichen Faktoren wirken auch auf Haare, wo es ebenfalls zu einer Schädigung kommen kann. Die Haare werden spröde, weniger elastisch und glanzlos. Die Oberflächenstruktur der Haare ist geschädigt.

Kosmetische oder dermatologische Pflegeprodukte mit Eigenschaften, die den beschriebenen oder vergleichbaren Prozessen entgegenwirken oder deren schädliche Folgen mindern oder rückgängig machen sollen, zeichnen sich häufig durch folgende spezifische Eigenschaften aus - radikalfangend, antioxidativ, entzündungshemmend oder feuchthaltend wirksam. Sie verhindern oder reduzieren u.a. die Aktivität der matrixabbauenden Enzyme oder regulieren die Neusynthese von Kollagen, Elastin oder Proteoglycanen.

Die Verwendung von Antioxidantien oder Radikalfängern in kosmetischen Zubereitungen ist an sich hinlänglich bekannt. So ist der Einsatz des antioxidativen Vitamin E in Sonnenschutzformulierungen üblich. Dennoch bleibt auch hier die erzielte Wirkung hinter der erhofften weit zurück.

Vitamin A und Vitamin-A-Derivate, wie Retinsäure, Retoinol und Retinol-Ester, wirken auf die Differenzierung von Epithelzellen und werden daher zur Prophylaxe und Behandlung zahlreicher den Hautzustand beeinträchtigender Phänomene eingesetzt; z.B. ist die Verwendung gegen Akne, Psoriaris, Altersflecken, Hautverfärbungen und Falten beschrieben. (vgl. z.B. WO 93/19743, WO 02/02074).

Allerdings wird auch eine hautirritierende Wirkung von Retinol und Derivaten in der Literatur beschrieben (z.B. WO 94/07462). Durch diese Nebenwirkungen ist die Anwendung von Retinol auf eng begrenzte Bereiche beschränkt, wobei eine Überdosierung vermieden werden muss. Es besteht daher Bedarf nach Wirkstoffen, die ein Retinol-ähnliches Wirkspektrum aufweisen, die beschriebenen Nebenwirkungen jedoch nicht oder zumindest nur in verminderter Form aufweisen.

Aufgrund des immer größer werdenden Bedarfs an Wirkstoffen zur vorbeugenden Behandlung von menschlicher Haut und menschlicher Haare gegen Alterungsprozesse und schädigenden Umwelteinflüssen war es Aufgabe der vorliegenden Erfindung, neue Wirkstoffe bereitzustellen, die die bereits eingangs genannten Wirkungen zeigen, ausreichend oxidations- und photostabil sowie gut formulierbar sein sollen. Die damit hergestellten Zubereitungen sollen ferner möglichst ein niedriges Irritationspotential für die Haut aufweisen, sie sollen möglichst die Wasserbindung in der Haut positiv beeinflussen, die Elastizität der Haut erhalten oder erhöhen und somit eine Glättung der Haut fördern. Darüber hinaus sollen sie vorzugsweise beim Auftragen auf die Haut ein angenehmes Hautgefühl erzeugen.

Jetzt wurde überraschend gefunden, dass sich bestimmte Chromen-2-on-Derivate (Chromon-Derivate) sich als Wirkstoffe mit dem beschriebenen Profil eignen.

Denkbar ist die Verwendung zumindest einer Verbindung der Formel I

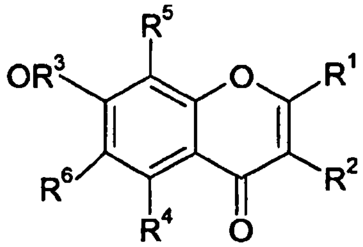

I

oder einer Zubereitung enthaltend mindestens eine Verbindung der Formel I, wobei
$R^1$ und $R^2$ gleich oder verschieden sein können und ausgewählt sind aus

- H, -C(=O)-$R^7$, -C(=O)-$OR^7$,
- geradkettigen oder verzweigten $C_1$- bis $C_{20}$-Alkylgruppen,
- geradkettigen oder verzweigten $C_3$- bis $C_{20}$-Alkenylgruppen, geradkettigen oder verzweigten $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder.

    - $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können,

$R^3$ steht für H oder geradkettige oder verzweigte $C_1$- bis $C_{20}$-Alkylgruppen,
$R^4$ steht für H oder $OR^8$,
$R^5$ und $R^6$ gleich oder verschieden sein können und ausgewählt sind aus

- -H, -OH,
- geradkettigen oder verzweigten $C_1$- bis $C_{20}$-Alkylgruppen,
- geradkettigen oder verzweigten $C_3$- bis $C_{20}$-Alkenylgruppen,
- geradkettigen oder verzweigten $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann und

$R^7$ steht für H, geradkettige oder verzweigte $C_1$- bis $C_{20}$-Alkylgruppen, eine Polyhydroxy-Verbindung, wie vorzugsweise einen Ascorbinsäurerest oder glycosidische Reste und
$R^8$ steht für H oder geradkettige oder verzweigte $C_1$- bis $C_{20}$-Alkylgruppen, wobei mindestens 2 der Substituenten $R^1$, $R^2$, $R^4$-$R^6$ verschieden von H sind oder mindestens ein Substituent aus $R^1$ und $R^2$ für -C(=O)-$R^7$ oder - C(=O)-$OR^7$ steht, zur Pflege, Konservierung oder Verbesserung des allgemeinen Zustandes der Haut oder Haare.

Ein erster Gegenstand der vorliegenden Erfindung ist die nichttherapeutische Verwendung der Verbindung der Formel Ia

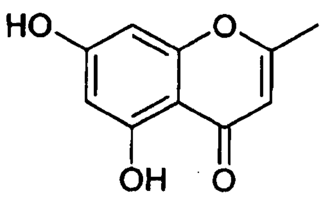

Ia

oder einer Zubereitung enthaltend die Verbindung der Formel Ia zur Prophylaxe gegen oder Reduktion von Hautunebenheiten.

Grundsätzlich sind im Sinne der vorliegenden Erfindung von der Bezeichnung Verbindung nach Formel Ia auch die Salze der Verbindungen nach Formel Ia umfasst. Zu den bevorzugten Salzen gehören dabei insbesondere Alkali- und Erdalkalimetallsalze sowie AmmoniumSalze, insbesondere jedoch Natrium- und Kalium-Salze.

Ein weiterer Gegenstand der vorliegenden Erfindung sind Zubereitung enthaltend mindestens eine Verbindung gemäß Formel Ia mit Resten wie oben definiert, sowie mindestens einen weiteren hautpflegenden Inhaltsstoff und mindestens einen für topische Anwendungen geeigneten Träger.

Erfindungsgemäß bevorzugte Verwendungen der Verbindungen gemäß Formel Ia bzw. von Zubereitungen

enthaltend mindestens eine Verbindung nach Formel Ia sind dabei insbesondere die Verwendung zur Prophylaxe gegen zeit- und/oder lichtinduzierte Alterungsprozesse der menschlichen Haut oder menschlicher Haare, insbesondere zur Prophylaxe gegen trockene Haut, Faltenbildung und/oder Pigmentstörungen, und/oder zur Reduktion oder Verhinderung schädigender Effekte von UV-Strahten auf die Haut, sowie zur Prophylaxe gegen oder Reduktion von Hautunebenheiten, wie Falten, feinen Linien, rauher Haut oder großporiger Haut.

Erfindungsgemäß bevorzugte nicht-therapeutische Verwendungen der Verbindungen gemäß Formel Ia bzw. von Zubereitungen enthaltend die Verbindung nach Formel Ia sind weiter die Verwendung zur Prophylaxe und/oder Verhinderung von vorzeitiger Hautalterung, insbesondere zur Prophylaxe und/oder Verhinderung von licht- oder alterungsbedingter Faltenbildung der Haut, zur Verminderung der Pigmentierungen und der Keratosis actinica und zur Prophylaxe und/oder Behandlung aller Krankheiten, die mit der normalen Alterung oder der licht-bedingten Alterung der Haut zusammenhängen, sowie zur Prophylaxe und/oder Behandlung von Hautkrankheiten, die mit einer Störung der Keratinisierung verbunden sind, die die Differenzierung und Zellprolif-eration betrifft, insbesondere zur Behandlung der Akne vulgaris, Akne comedonicä, der polymorphen Akne, der Akne rosaceae, der nodulären Akne, der Akne conglobata, der alters-bedingten Aknen, der als Nebenwirkung auftretenden Aknen, wie der Akne solaris, der medikamenten-bedingten Akne oder der Akne professionalis, zur Behandlung anderer Störungen der Keratinisierung, insbesondere der Ichtyosen, der ichtyosi-formen Zustände, der Darrier-Krankheit, der Keratosis palmoplantaris, der Leukoplasien, der leukoplasiformen Zustände, der Haut- und Schleimhaut-flechten (Buccal) (Lichen), zur Behandlung anderer Hauterkrankungen, die mit einer Störung der Keratinisierung zusammenhängen und eine entzünd-liche und/oder immunoallergische Komponente haben und insbesondere aller Formen der Psoriasis, die die Haut, die Schleimhäute und die Finger und Zehennägel betreffen, und des psoriatischen Rheumas und der Hautatopien, wie Ekzemen oder der respiratorischen Atopie oder auch der Hypertrophie des Zahnfleisches, sowie zur Prophylaxe und/oder Behandlung aller gutartigen oder bösartigen Wucherungen der Dermis oder Epidermis, die gegebenenfalls viralen Ursprungs sind, wie Verruca vulgaris. Veruca plana, Epidermodysplasia verruciformis, orale Papillomatose, Papillo-matosis florida, und der Wucherungen, die durch UV-Strahlung hervor-gerufen werden können, insbesondere des Epithelioma baso-cellulare und Epithelioma spinocellulare.

Dabei ist jeweils die Verwendung der Verbindungen gemäß Formel Ia zur Herstellung von Zubereitungen geeignet zu den oben angegebenen Verwendungen auch Gegenstand der vorliegenden Erfindung.

Bei den Zubereitungen handelt es sich dabei üblicherweise entweder um topisch anwendbare Zubereitungen, beispielsweise kosmetische oder dermatologische Formulierungen, oder um Nahrungsmittel bzw. Nahrungsergänzungsmittel. Die Zubereitungen enthalten in diesem Fall einen kosmetisch oder dermatologisch oder Nahrungsmittel-geeigneten Träger und je nach gewünschtem Eigenschaftsprofil optional weitere geeignete Inhaltsstoffe.

Die erfindungsgemäße Verwendung von Chromen-4-on-Derivaten der Formel Ia in Zubereitungen bietet u.a. einen Schutz vor Schäden, die durch UV-Strahlung oder durch reaktive Verbindungen hervorgerufene Prozesse direkt oder indirekt verursacht werden, wie z. B. der Hautalterung, dem Verlust der Hautfeuchtigkeit, dem Verlust der Hautelastizität, der Bildung von Falten oder Runzeln oder von Pigmentstörungen oder Altersflecken.

Weiterhin betrifft die vorliegende Erfindung die Verwendung der o.g. Zubereitungen zur Vorbeugung unerwünschter Veränderungen des Hautbildes, wie z.B. Akne oder fettige Haut, Keratosen, lichtempfindliche, entzündliche, erythrematöse, allergische oder autoimmunreaktive Reaktionen.

Die erfindungsgemäßen Verbindungen bzw. Zubereitungen dienen vorzugsweise aber auch zur Beruhigung von empfindlicher und gereizter Haut, zur vorbeugenden Regulation der Kollagen-, Hyaluronsäure-, Elastinsynthese, Stimulation der DNA-Synthese, insbesondere bei defizitären oder hypoaktiven Hautzuständen, Regulation der Transkription und Translation matrixabbauender Enzyme, insbesondere der MMPs, Steigerung der Zellerneuerung und Regeneration der Haut, Steigerung der hauteigenen Schutz- und Reparaturmechanismen für DNA, Lipide und/oder Proteine.

Verbindungen gemäß Formel I sind dadurch gekennzeichnet, dass $R^3$ steht für H und $R^4$ steht für OH da das Wirkpotential von Vertreten dieser Erfindungsklasse im oben genannten Sinne besonders hoch ist. Wenn zusätzlich mindestens einer der Reste $R^5$ und $R^6$ für OH steht, verfügen diese bevorzugten Verbindungen neben den oben genannten Eigenschaften zusätzlich über ein antioxidantes Potential. Daher können sie in Zubereitungen gleichzeitig als Antioxidans fungieren.

Andere Verbindungen nach Formel I sind dadurch gekennzeichnet, dass $R^5$ und $R^6$ für H stehen. In diesem Fall sind die Reste $R^3$ und $R^4$ frei zugänglich, was, wie vermutet wird, vorteilhaft für die Interaktion mit an den genannten Wirkungen beteiligten Enzymen ist.

Weitere Verbindungen nach Formel I sind dadurch gekennzeichnet, dass einer der Reste $R^1$ oder $R^2$ für H steht und der andere Rest steht für $-C(=O)-R^7$, $-C(=O)-OR^7$ oder eine geradkettige oder verzweigte $C_1$- bis $C_{20}$-Alkylgruppe.

Zusätzlich haben die Verbindungen Vorteile bei der Einarbeitung in die Zubereitungen:

- Mono- und/oder Oligoglycosylreste verbessern die Wasserlöslichkeit der erfindungsgemäß einzusetzenden Ver-

bindungen;

- geradkettige oder verzweigte $C_1$- bis $C_{20}$-Alkoxygruppen, insbesondere die langkettigen Alkoxyfunktionen, wie Ethylhexyloxy-Gruppen erhöhen die Öllöslichkeit der Verbindungen;

d.h. über die geeignete Auswahl der Substituenten kann die Hydrophilie bzw. Lipophilie der erfindungsgemäßen Verbindungen gesteuert werden.

Als glycoside Reste können insbesondere Mono- oder Oligosaccharidreste eingesetzt werden. Bevorzugt sind dabei Hexosylreste, insbesondere Ramnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch vorteilhaft sein, Pentosylreste zu verwenden. Die Glycosylreste können $\alpha$-oder $\beta$-glycosidisch mit dem Grundkörper verbunden sein. Ein bevorzugtes Disaccharid ist beispielsweise das 6-O-(6-deoxy-$\alpha$-L-mannopyranosyl)-$\beta$-D-glucopyranosid.

Die erfindungsgemäßen Zubereitungen können in ebenfalls bevorzugten Ausführungsformen der Erfindung jedoch auch in der Zubereitungs-Matrix schlecht oder nicht lösliche Verbindungen der Formel Ia enthalten. In diesem Fall liegen die Verbindungen vorzugsweise in feinteiliger Form in der kosmetischen Zubereitung dispergiert vor.

Insbesondere ist die Verwendung von Verbindungen ausgewählt aus den Verbindungen mit den Formeln Ia-In möglich:

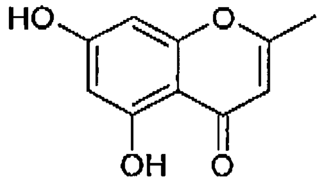

Ia

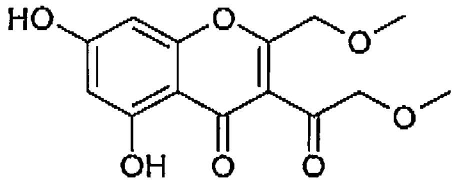

Ib

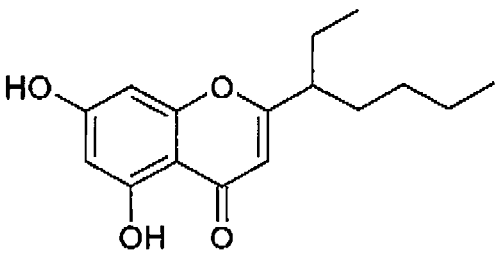

Ic

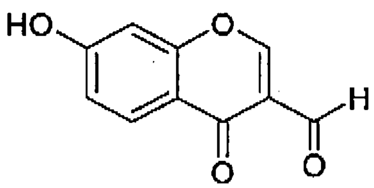

Id

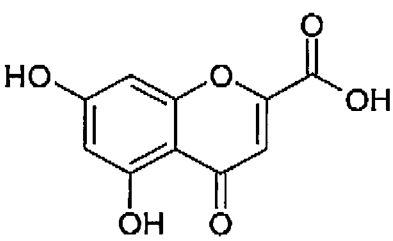

Ie

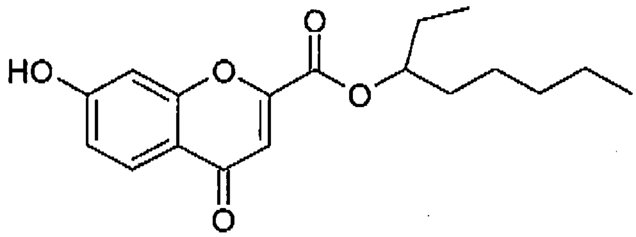

If

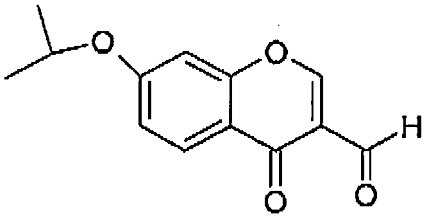

Ig

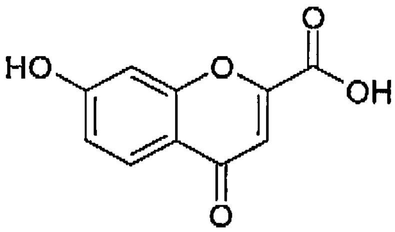

Ih

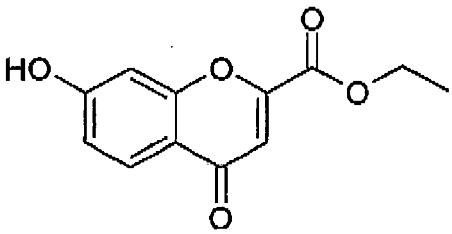

Ii

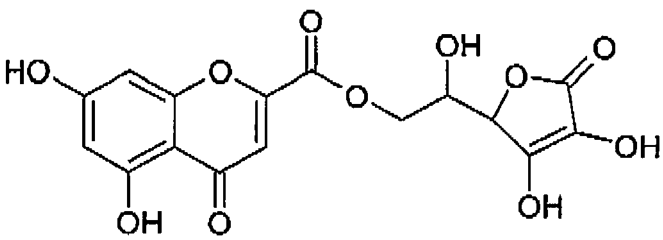

Ik

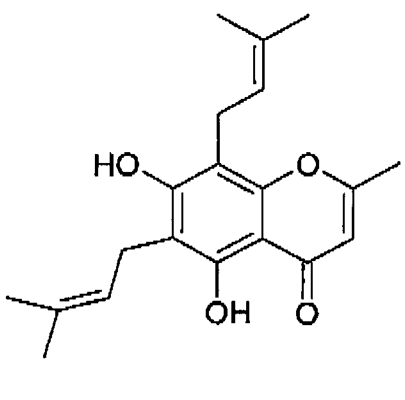

Im

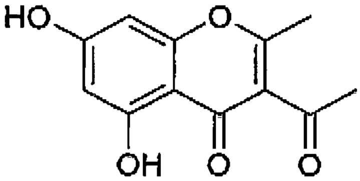

In

Anwendungen strukturell verwandter Verbindungen sind aus der Literatur bekannt:

Die Verwendung von bestimmten 2-(Alkyl)carboxyl oder 2-(Alkyl)phenyl substituierten Chromen-4-onderivaten in Kombination mit zweiwertigem Zink in pharmazeutischen und kosmetischen Zubereitungen ist aus EP-A-0 304 802 bekannt. Die Zubereitungen eignen sich zur Hautbehandlung, insbesondere zur Behandlung von Dermatosen einschließlich atopischem Ekzem.

Aus EP-A-0 424 444 ist die Verwendung von Salzen der Chromoncarboxylsäure in Kosmetika zur Bekämpfung der Hautalterung bekannt. Dabei zeigt die Verbindung eine UV-filternde Wirkung und hat im Tierversuch folgende Wirkungen: der Anteil gebundener Lipide in der Haut erhöht sich, der Anteil an löslichen Kollagen in der Haut wird erhöht, die Widerstandsfähigkeit der Haut gegenüber einwirkungen der fibroplatischen Proteasen Kollagenase und Elastase wird erhöht.

Aus US 6,019,992 sind kosmetische Zubereitungen bekannt, die 4-Chromanon enthalten, und die sich zur Behandlung von gealterter, trockener oder faltiger Haut eignen. Dabei wird gezeigt, dass 4-Chromanon in Keratinozyten-Kulturen die Differenzierung der Zellen fördert und die Lipid-Produktion anregt.

Aus EP-A-1 216 692 ist die Verwendung von 2-Methyl-2-(β-Carboxyethyl)-Chroman-Derivaten in kosmetischen Zubereitungen bekannt. Die genannten Zubereitungen eignen sich besonders zur Prophylaxe gegen Alterungsprozesse von Haut und Haaren sowie zur Prophylaxe gegen trockene Haut, Faltenbildung und Pigmentstörungen.

Zubereitungen zur topischen Anwendung, die Chromon-Derivate, wie z.B. Chromon, 7-Hydroxychromon, 7-Methoxychromon, 5,7-Dihydroxy-2-methyl-chromon, 3-Methyl -2-butenyloxy-chromon, 3-Acetyl-5,7-dihydroxy-2-methyl-chromon, 5-hydroxychromon, n-Pentyl-7-methoxychromon-2-carboxylat, n-Undecyl-5-methoxychromon-2-carboxylat, 5-Hydroxy-7-methoxy-2-methyl-chromon, 7-Methoxy-chromon-2-carbonsäure, n-Pentyl-chromon-2-carbonsäure, 5-methoxychromon und Chromon-2-carbonsäure, enthalten, sind aus der japanischen Patentanmeldung JP 05/301813 bekannt. Die Chromon-Derivate wirken als hautverträgliche Tyrosinase-Inhibitoren, welche die Hyperpigmentierung der Haut verringern.

Aus der Japanischen Patentanmeldung JP 09/188608 ist die Verwendung von substituierten Chromon-Derivaten, wie insbesondere 5,7-Dihydroxychromone, 7-Methoxychromone, 5-Hydroxy-7-methoxy-2-methyl-chromon und 5-Hydroxy-2-methyl-chromon, als Wirkstoff gegen graue Haare bekannt. Die Wirkung wird dabei auf die Aktivierung der Farbpigment-bildenden Zellen und die Steigerung der Melanogenese zurückgeführt.

Ein Mittel gegen Hautalterung enthaltend Chromon-Derivate, die in Position 2 mit $C_{1-15}$-Alkyl substituiert sind und in Position 7 H-, OH- oder Alkoxy-Substitution aufweisen, in Kombination mit Aminopropanolderivaten ist aus JP 10/194919 bekannt.

Kosmetische Zubereitung, die substituierte Chromon-Derivaten, wie z.B. 2-(1-Ethylpentyl)-chromon, 5,7-Dihydroxychromone, 7-Methoxychromone, 5-Hydroxy-7-methoxy-2-methyl-chromon und 5-Hydroxy-2-methyl-chromon und aromatische Verbindungen mit einem Schmelzpunkt von -10°C oder darüber enthalten, sind aus JP 10/114640 bekannt. Dabei erleichtert das Chromon-Derivat die Einarbeitung der aromatischen Verbindung in die kosmetische Formulierung.

Die Verbindungen der Formel Ia werden erfindungsgemäß typisch in Mengen von 0,01 bis 20 Gew.-%, vorzugsweise in Mengen von 0,1 Gew.-% bis 10 Gew.-% und insbesondere bevorzugt in Mengen von 1 bis 8 Gew.-% eingesetzt. Dabei bereitet es dem Fachmann keinerlei Schwierigkeiten die Mengen abhängig von der beabsichtigten Wirkung der Zubereitung entsprechend auszuwählen.

Die schützende Wirkung gegen oxidativen Stress bzw. gegen die Einwirkung von Radikalen kann also weiter verbessert werden, wenn die Zubereitungen ein oder mehrere weitere Antioxidantien enthalten, wobei es dem Fachmann keinerlei Schwierigkeiten bereitet geeignet schnell oder zeitverzögert wirkende Antioxidantien auszuwählen..

In einer bevorzugten Ausführungsform der vorliegenden Erfindungen handelt es sich bei mindestens einem weiteren hautpflegenden Inhaltsstoff um ein oder mehrere Antioxidantien und/oder Vitamine.

Dabei ist es aus den oben genannten Gründen insbesondere bevorzugt, wenn die Zubereitung kein Retinol-Derivat enthält.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Camosin, D-Carnosin, L-Carnosin und deren Derivate (z.B. Anserin), Carotinoide, Carotine (z.B, α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, $ZnSO_4$), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex® AP), natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004). Derartige Antioxidantien werden mit Verbindungen der Formel I in solchen Zusammensetzungen überlicherweise in Verhältnissen im Bereich von 1000:1 bis 1:1000, bevorzugt in Mengen von 100:1 bis 1:100 eingesetzt.

Die erfindungsgemäßen Zubereitungen können als weitere Inhaltsstoffe Vitamine enthalten. Bevorzugt sind Vitamine und Vitamin-Derivate ausgewählt aus Vitamin B, Thiaminchloridhydrochlorid (Vitamin $B_1$), Riboflavin (Vitamin $B_2$), Nicotinsäureamid, Vitamin C (Ascorbinsäure), Vitamin D, Ergocalciferol (Vitamin $D_2$), Vitamin E, DL-α-Tocopherol, Tocopherol-E-Acetat, Tocopherolhydrogensuccinat, Vitamin $K_1$, Esculin (Vitamin P-Wirkstoff), Thiamin (Vitamin $B_1$), Nicotinsäure (Niacin), Pyridoxin, Pyridoxal, Pyridoxamin, (Vitamin $B_6$), Panthothensäure, Biotin, Folsäure und Cobalamin (Vitamin $B_{12}$) in den erfindungsgemäßen kosmetischen Zubereitungen enthalten, insbesondere bevorzugt Vitamin C und dessen Derivaten, DL-α-Tocopherol, Tocopherol-E-Acetat, Nicotinsäure, Pantothensäure und Biotin. Vitamine werden dabei mit Verbindungen der Formel I überlicherweise in Verhältnissen im Bereich von 1000:1 bis 1:1000, bevorzugt in Mengen von 100:1 bis 1:100 eingesetzt.

Unter den Phenolen mit antioxidativer Wirkung sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydoxyflavonen beschäftigen sich K. Lemanska, H. Szymusiak, B. Tyrakowska, R. Zielinski, I.M.C.M. Rietjens; Current Topics in Biophysics 2000, 24(2), 101-108. Es wird dort beobachtet, dass Dihydroxyflavone mit einer OH-Gruppe benachbart zur Ketofunktion oder OH-Gruppen in 3'4'- oder 6,7- oder 7,8-Position antioxidative Eigenschaften aufweisen, während andere Mono- und Dihydroxyflavone teilweise keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers, I.M.C.M. Rietjens; Free Radical Biology&Medicine 2001, 31 (7), 869-881 untersuchen die pH-Abhängigkeit der antioxidanten Wirkung von Hydoxyflavonen. Über den gesamten pH-Bereich zeigt Quercetin die höchste Aktivität der untersuchten Strukturen.

Geeignete Antioxidantien sind weiter Verbindungen der Formel II

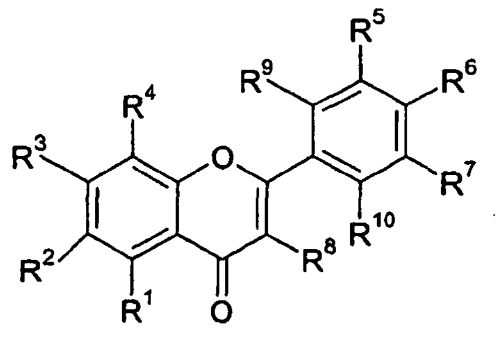

II

wobei $R^1$ bis $R^{10}$ gleich oder verschieden sein können und ausgewählt sind aus

- H
- $OR^{11}$
- geradkettigen oder verzweigten $C_1$- bis $C_{20}$-Alkylgruppen,
- geradkettigen oder verzweigten $C_3$- bis $C_{20}$-Alkenylgruppen,
- geradkettigen oder verzweigten $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können,
- wobei alle $OR^{11}$ unabhängig voneinander stehen für
    - OH
    - geradkettige oder verzweigte $C_1$- bis $C_{20}$-Alkyloxygruppen,
    - geradkettigen oder verzweigten $C_3$- bis $C_{20}$-Alkenyloxygruppen,
    - geradkettigen oder verzweigten $C_1$- bis $C_{20}$-Hydroxyalkoxygruppen, wobei die Hydroxygruppe(n) an ein primäre oder sekundäre Kohlenstoffatome der Kette gebunden sein können und weiter die Alkylkette auch durch Sauerstoff unterbrochen sein kann, und/oder
    - $C_3$- bis $C_{10}$-Cycloalkyloxygruppen und/oder $C_3$- bis $C_{12}$-Cycloalkenyloxygruppen, wobei die Ringe jeweils auch durch $-(CH_2)_n$-Gruppen mit n = 1 bis 3 überbrückt sein können und/oder,
    - Mono- und/oder Oligoglycosylreste,

mit der Maßgabe, dass mindestens 4 Reste aus $R^1$ bis $R^7$ stehen für OH und dass im Molekül mindestens 2 Paare benachbarter Gruppen -OH vorliegen,

- oder $R^2$, $R^5$ und $R^6$ für OH und die Reste $R^1$, $R^3$, $R^4$ und $R^{7-10}$ für H stehen,

wie sie in der älteren Deutschen Patentanmeldung DE 10244282.7 beschrieben sind.

Vorteile der erfindungsgemäßen Zusammensetzungen enthaltend mindestens ein Antioxidans sind dabei neben den oben genannten Vorteilen insbesondere die antioxidante Wirkung und die gute Hautverträglichkeit. Zusätzlich sind bevorzugte der hier beschriebenen Verbindungen farblos oder nur schwach gefärbt und führen so nicht oder nur in geringer Weise zu Verfärbungen der Zubereitungen. Von Vorteil ist insbesondere das besondere Wirkprofil der Verbindungen nach Formel II, welches sich im DPPH-Assay in einer hohen Kapazität Radikale zu fangen ($EC_{50}$), einer zeitverzögerten Wirkung ($T_{EC50}$ > 120 min) und damit einer mittleren bis hohen antiradikalischen Effizienz (AE) äußert. Zudem vereinigen die Verbindungen nach Formel II im Molekül antioxidative Eigenschaften mit UV-Absorption im UV-A- und/oder -B-Bereich. Bevorzugt sind daher auch Zubereitungen enthaltend zumindest eine Verbindung der Formel II, die dadurch gekennzeichnet ist, dass mindestens zwei benachbarte Reste der Reste $R^1$ bis $R^4$ stehen für OH und mindestens zwei benachbarte Reste der Reste $R^5$ bis $R^7$ stehen für OH. Insbesondere bevorzugte Zubereitungen enthalten zumindest eine Verbindung der Formel II, die dadurch gekennzeichnet ist, dass mindestens drei benachbarte Reste der Reste $R^1$ bis $R^4$ stehen für OH, wobei vorzugsweise die Reste $R^1$ bis $R^3$ für OH stehen.

Damit die Verbindungen der Formel Ia ihre positive Wirkung auf die Haut besonders gut entwickeln können, kann es bevorzugt sein die Verbindungen der Formel Ia in tiefere Hautschichten eindringen zu lassen. Dazu stehen mehrere Möglichkeiten zur Verfügung. Zum einen können die Verbindungen der Formel Ia eine ausreichende Lipophilie aufweisen, um durch die äußere Hautschicht in epidermale Schichten vordringen zu können. Als weitere Möglichkeit können in der Zubereitung auch entsprechende Transportmittel, beispielsweise Liposomen, vorgesehen sein, die einen

Transport der Verbindungen der Formel Ia durch die äußeren Hautschichten ermöglichen. Schließlich ist auch ein systemischer Transport der Verbindungen der Formel Ia denkbar. Die Zubereitung wird dann beispielsweise so gestaltet, dass sie für eine orale Gabe geeignet ist.

Es ist auch vorteilhaft, die Verbindungen der Formel II in verkapselter Form darzureichen, z. B. als Cellulose- oder Chitin-kapseln, in Gelatine bzw. Wachsmatrices oder mit Cyclodextrinen verkapselt.

Es wird vermutet, dass die Verbindung der Formel Ia auch als Enzymhemmer wirken. Sie hemmen vermutlich Proteinkinasen, Elastase, Aldosereduktase sowie Hyaluronidase, und ermöglichen daher, die Unversehrtheit der Grundsubstanz vaskulärer Hüllen aufrecht zu erhalten. Ferner hemmen sie vermutlich nicht spezifisch Katechol-O-methyltransferase, wodurch die Menge der verfügbaren Katecholamine und dadurch die Gefäßfestigkeit erhöht wird. Weiter hemmen sie vermutlich die AMP-Phosphodiesterase, wodurch die Substanzen ein Potential zur Hemmung der Thrombozytenaggregation aufweisen.

Aufgrund dieser Eigenschaften eignen sich die erfindungsgemäßen Zubereitungen allgemein zur Immunprotektion und zum Schutz der DNA und RNA. Insbesondere eignen sich die Zubereitungen dabei zum Schutz von DNA und RNA vor oxidativen Angriffen, vor Radikalen und vor Schädigung durch Strahlung, insbesondere UV-Strahlung. Ein weiterer Vorteil der erfindungsgemäßen Zubereitungen ist der Zellschutz, insbesondere der Schutz von Langerhans-Zellen vor Schäden durch die oben genannten Einflüsse. Alle diese Verwendungen bzw. die Verwendung der Verbindungen der Formel Ia zur Herstellung entsprechend einsetzbarer Zubereitungen sind ausdrücklich auch Gegenstand der vorliegenden Erfindung.

Insbesondere eignen sich bevorzugte erfindungsgemäße Zusammensetzungen auch zur Behandlung von Hautkrankheiten, die mit einer Störung der Keratinisierung verbunden sind, die die Differenzierung und Zellprolif-eration betrifft, insbe-sondere zur Behandlung der Akne vulgaris, Akne comedonicä, der polymorphen Akne, der Akne rosaceae, der nodulären Akne, der Akne conglobata, der alters-bedingten Aknen, der als Neben-wirkung auftretenden Aknen, wie der Akne solaris, der medikamenten-bedingten Akne oder der Akne professionalis, zur Behandlung anderer Störungen der Keratinisierung, insbesondere der Ichtyosen, der ichtyosi-formen Zustände, der Darrier-Krankheit, der Keratosis palmoplantaris, der Leukoplasien, der leukoplasiformen Zustände, der Haut- und Schleimhaut-flechten (Buccal) (Lichen), zur Behandlung anderer Hauterkrankungen, die mit einer Störung der Keratinisierung zusammenhängen und eine entzünd-liche und/oder immunoallergische Komponente haben und insbesondere aller Formen der Psoriasis, die die Haut, die Schleimhäute und die Finger und Zehennägel betreffen, und des psoriatischen Rheumas und der Hautatopien, wie Ekzemen oder der respiratorischen Atopie oder auch der Hypertrophie des Zahnfleisches, wobei die Verbindungen ferner bei einigen Entzündungen verwendet werden können, die nicht mit einer Störung der Keratinisierung zusammenhängen, zur Behandlung aller gutartigen oder bösartigen Wucherungen der Dermis oder Epidermis, die gegebenenfalls viralen Ursprungs sind, wie Verruca vulgaris. Veruca plana, Epidermodysplasia verruciformis, orale Papillomatose, Papillo-matosis florida, und der Wucherungen, die durch UV-Strahlung hervorgerufen werden können, insbesondere des Epithelioma baso-cellulare und Epithelioma spinocellulare, zur Behandlung anderer Hautkrankheiten, wie der Dermatitis bullosa und der das Kollagen betreffenden Krankheiten, zur Behandlung bestimmter Augenkrankheiten, insbesondere der Hornhauterkran-kungen, zur Behebung oder Bekämpfung der lichtbedingten und der mit dem Älterwerden zusammenhängenden Hautalterung, zur Verminderung der Pigmentierungen und der Keratosis actinica und zur Behandlung aller Krankheiten, die mit der normalen Alterung oder der lichtbedingten Alterung zusammenhängen, zur Vorbeugung vor oder der Heilung von Wunden/Narben der Atrophien der Epidermis und/oder Dermis, die durch lokal oder systemisch angewendete Corticosteroide hervorgerufen werden und aller sonstigen Arten der Hautatrophie, zur Vorbeugung vor oder Behandlung von Störungen der Wundheilung, zur Vermeidung oder Behebung von Schwangerschaftsstreifen oder auch zur Förderung der Wundheilung, zur Bekämpfung von Störungen der Talgproduktion, wie Hypersebhorrhö bei Akne oder der einfachen Seborrhö, zur Bekämpfung von oder Vorbeugung von krebsartigen Zuständen oder vor präkanzerogenen Zuständen, insbesondere der promyelozytären Leukämien, zur Behandlung von Entzündungserkrankungen, wie Arthritis, zur Behandlung aller virusbedingten Erkrankungen der Haut oder anderer Bereiche des Körpers, zur Vorbeugung vor oder Behandlung der Alopecie, zur Behandlung von Hautkrankheiten oder Krankheiten anderer Körperbereiche mit einer immuno-logischen Komponente, zur Behandlung von Herz-/Kreislauf-Erkran-kungen, wie Arteriosklerose oder Bluthochdruck, sowie des Insulin-unabhängigen Diabetes, zur Behandlung von Hautproblemen, die durch UV-Strahlung hervorgerufen werden.

Erfindungsgemäß insbesondere bevorzugte Zubereitungen enthalten neben den Verbindungen der Formel Iauch UV-Filter.

Bei Einsatz der als UV-A-Filter insbesondere bevorzugten Dibenzoylmethanderivate in Kombination mit den Verbindungen der Formel Ia ergibt sich ein zusätzlicher Vorteil: Die UV-empfindlichen Dibenzoylmethanderivate werden durch die Anwesenheit der Verbindungen der Formel Ia zusätzlich stabilisiert. Ein weiterer Gegenstand der vorliegenden Erfindung ist daher die Verwendung der Verbindungen gemäß Formel Ia zur Stabilisierung von Dibenzoylmethanderivaten in Zubereitungen.

Prinzipiell kommen alle UV-Filter für eine Kombination mit den erfindungsgemäßen Verbindungen der Formel Ia in Frage. Besonders bevorzugt sind solche UV-Filter, deren physiologische Unbedenklichkeit bereits nachgewiesen

ist. Sowohl für UVA wie auch UVB-Filter gibt es viele aus der Fachliteratur bekannte und bewährte Substanzen, z.B. Benzylidenkampferderivate wie 3-(4'-Methylbenzyliden)-dl-kampfer (z.B. Eusolex® 6300), 3-Benzylidenkampfer (z.B. Memoryl® SD), Polymere von N-{(2 und 4)-[(2-oxobom-3-yliden)methyl]benzylfacrylamid (z.B. Mexoryl® SW), N,N,N-Trimethyl-4-(2-oxoborn-3-ylidenmethyl)anilinium methylsulfat (z.B. Mexoryl® SK) oder (2-Oxoborn-3-yliden)toluol-4-sulfonsäure (z.B. Mexoryl® SL),
Benzoyl- oder Dibenzoylmethane wie 1-(4-tert-Butylphenyl)-3-(4-methoxyphenyl)propan-1,3-dion (z.B. Eusolex® 9020) oder 4-Isopropyldibenzoylmethan (z.B. Eusolex® 8020),
Benzophenone wie 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40),
Methoxyzimtsäureester wie Methoxyzimtsäureoctylester (z.B. Eusolex® 2292), 4-Methoxyzimtsäureisopentylester, z.B. als Gemisch der Isomere (z.B. Neo Heliopan® E 1000),
Salicylatderivate wie 2-Ethylhexylsalicylat (z.B. Eusolex® OS), 4-Isopropylbenzylsalicylat (z.B. Megasol®) oder 3,3,5-Trimethylcyclohexylsalicylat (z.B. Eusolex® HMS),
4-Aminobenzoesäure und Derivate wie 4-Aminobenzoesäure, 4-(Dimethylamino)benzoesäure-2-ethylhexylester (z.B. Eusolex® 6007), ethoxylierter 4-Aminobenzoesäureethylester (z.B. Uvinul® P25),
Phenylbenzimidazolsulfonsäuren, wie 2-Phenylbenzimidazol-5-sulfonsäure sowie ihre Kalium-, Natrium- und Triethanolaminsalze (z.B. Eusolex® 232), 2,2-(1,4-Phenylen)-bisbenzimidazol-4,6-disulfonsäure bzw. deren Salze (z.B. Neoheliopan® AP) oder 2,2-(1,4-Phenylen)-bisbenzimidazol-6-sulfonsäure;
und weitere Substanzen wie

- 2-Cyano-3,3-diphenylacrylsäure-2-ethylhexylester (z.B. Eusolex® OCR),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX) und
- 2,4,6-Trianilino-(p-carbo-2'-ethylhexyl-l'-oxi)-1,3,5-triazin (z.B. Uvinul® T 150)
- 2-(4-Diethylamino-2-hydroxy-benzoyl)-benzoesäure hexylester (z.B. Uvinul®UVA Plus, Fa. BASF).

Die in der Liste aufgeführten Verbindungen sind nur als Beispiele aufzufassen. Selbstverständlich können auch andere UV-Filter verwendet werden.
Diese organischen UV-Filter werden in der Regel in einer Menge von 0,5 bis 10 Gewichtsprozent, vorzugsweise 1 - 8 %, in kosmetische Formulierungen eingearbeitet.
Weitere geeignete organische UV-Filter sind z.B.

- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole®),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),
- $\alpha$-(Trimethylsilyl)-$\omega$-[trimethylsilyl)oxy]poly[oxy(dimethyl [und ca. 6% methyl[2-[p-[2,2-bis(ethoxycarbonyl]vinyl]phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] (n $\approx$ 60) (CAS-Nr. 207 574-74-1)
- 2,2'-Methylen-bis-(6-(2H-benzotriazol-2-yl)-4-(1,1,3,3-tetramethyl-butyl)phenol) (CAS-Nr. 103 597-45-1)
- 2,2'-(1,4-Pheny)en)bis-(1H-benzimidazol-4,6-disulfonsäure, Mononatriumsalz) (CAS-Nr. 180 898-37-7) und
- 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6).
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),

Weitere geeignete UV-Filter sind auch Methoxyflavone ensprechend der älteren Deutschen Patentanmeldung DE 10232595.2.
Organische UV-Filter werden in der Regel in einer Menge von 0,5 bis 20 Gewichtsprozent, vorzugsweise 1 - 15 %, in kosmetische Formulierungen eingearbeitet.
Als anorganische UV-Filter sind solche aus der Gruppe der Titandioxide wie z.B. gecoatetes Titandioxid (z.B. Eusolex® T-2000, Eusolex®T-AQUA), Zinkoxide (z.B. Sachtotec®), Eisenoxide oder auch Ceroxide denkbar. Diese anorganischen UV-Filter werden in der Regel in einer Menge von 0,5 bis 20 Gewichtsprozent, vorzugsweise 2 - 10 %, in kosmetische Zubereitungen eingearbeitet.
Bevorzugte Verbindungen mit UV-filternden Eigenschaften sind 3-(4'-Methylbenzyliden)-dl-kampfer, 1-(4-tert-Butylphenyl)-3-(4-methoxy-phenyl)-pro-pan-1,3-dion, 4-Isopropyldibenzoylmethan, 2-Hydroxy-4-methoxy-benzophenon, Methoxyzimtsäureoctylester, 3,3,5-Trimethyl-cyclo-hexyl-sali-cylat, 4-(Dimethylamino)benzoesäure-2-ethyl-hexylester, 2-Cyano-3,3-di-phenyl-acrylsäure-2-ethylhexylester, 2-Phenyl-benzimidazol-5-sulfonsäure sowie ihre Kalium-,

Natrium- und Triethanol-aminsalze.

Durch Kombination von Verbindungen der Formel Ia mit weiteren UV-Filtern kann die Schutzwirkung gegen schädliche Einwirkungen der UV-Strahlung optimiert werden.

Optimierte Zusammensetzungen können beispielsweise die Kombination der organischen UV-Filter 4'-Methoxy-6-hydroxyflavon mit 1-(4-tert-Butylphenyl)-3-(4-methoxyphenyl)propan-1,3-dion und 3-(4'-Methylbenzyliden)-dl-kampfer enthalten. Mit dieser Kombination ergibt sich ein Breitbandschutz, der durch Zusatz von anorganischen UV-Filtern, wie Titandioxid-Mikropartikeln noch ergänzt werden kann.

Alle genannten UV-Filter können auch in verkapselter Form eingesetzt werden. Insbesondere ist es von Vorteil organische UV-Filter in verkapselter Form einzusetzen. Im Einzelnen ergeben sich die folgende Vorteile:

- Die Hydrophilie der Kapselwand kann unabhängig von der Löslichkeit des UV-Filters eingestellt werden. So können beispielsweise auch hydrophobe UV-Filter in rein wässrige Zubereitungen eingearbeitet werden. Zudem wird der häufig als unangenehm empfundene ölige Eindruck beim Auftragen der hydrophobe UV-Filter enthaltenden Zubereitung unterbunden.
- Bestimmte UV-Filter, insbesondere Dibenzoylmethanderivate, zeigen in kosmetischen Zubereitungen nur eine verminderte Photostabilität. Durch Verkapselung dieser Filter oder von Verbindungen, die die Photostabilität dieser Filter beeinträchtigen, wie beispielsweise Zimtsäurederivate, kann die Photostabilität der gesamten Zubereitung erhöht werden.
- in der Literatur wird immer wieder die Hautpenetration durch organische UV-Filter und das damit verbundene Reizpotential beim direkten Auftragen auf die menschliche Haut diskutiert. Durch die hier vorgeschlagene Verkapselung der entsprechenden Substanzen wird dieser Effekt unterbunden.
- Allgemein können durch Verkapselung einzelner UV-Filter oder anderer Inhaltstoffe Zubereitungsprobleme, die durch Wechselwirkung einzelner Zubereitungsbestandteile untereinander entstehen, wie Kristallisationsvorgänge, Ausfällungen und Agglomeratbildung vermieden werden, da die Wechselwirkung unterbunden wird.

Daher ist es erfindungsgemäß bevorzugt, wenn ein oder mehrere der oben genannten UV-Filter in verkapselter Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben.

Geeignete Kapseln können Wände aus anorganischen oder organischen Polymeren aufweisen. Beispielsweise wird in US 6,242,099 B1 die Herstellung geeigneter Kapseln mit Wänden aus Chitin, Chitin-Derivaten oder polyhydroxylierten Polyaminen beschrieben. Erfindungsgemäß besonders bevorzugt einzusetzende Kapseln weisen Wände auf, die durch einen SolGel-Prozeß, wie er in den Anmeldungen WO 00/09652, WO 00/72806 und WO 00/71084 beschrieben ist, erhalten werden können. Bevorzugt sind hier wiederum Kapseln, deren Wände aus Kieselgel (Silica: undefiniertes Silicium-oxid-hydroxid) aufgebaut sind. Die Herstellung entsprechender Kapseln ist dem Fachmann beispielsweise aus den zitierten Patentanmeldungen bekannt, deren Inhalt ausdrücklich auch zum Gegenstand der vorliegenden Anmeldung gehört.

Dabei sind die Kapseln in erfindungsgemäßen Zubereitungen vorzugsweise in solchen Mengen enthalten, die gewährleisten, dass die verkapselten UV-Filter in den oben angegebenen Mengen in der Zubereitung vorliegen.

Bei den hautschonenden oder hautpflegenden Wirkstoffen kann es sich prinzipiell um alle den Fachmann bekannten Wirkstoffe handeln.

Besonders bevorzugte Wirkstoffe sind in einer Ausführungsform der vorliegenden Erfindung Pyrimidincarbonsäuren und/oder Aryloxime.

Pyrimidincarbonsäuren kommen in halophilen Mikroorganismen vor und spielen bei der Osmoregulation dieser Organismen eine Rolle (E. A. Galinski et al., Eur. J. Biochem., 149 (1985) Seite 135-139). Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure und deren Derivate zu nennen. Diese Verbindungen stabilisieren Enzyme und andere Biomoleküle in wässrigen Lösungen und organischen Lösungsmitteln. Weiter stabilisieren sie insbesondere Enzyme gegen denaturierende Bedingungen, wie Salze, extreme pH-Werte, Tenside, Harnstoff, Guanidiniumchlorid und andere Verbindungen.

Ectoin und Ectoin-Derivate wie Hydroxyectoin können vorteilhaft in Arzneimitteln verwendet werden. Insbesondere kann Hydroxyectoin zur Herstellung eines Arzneimittels zur Behandlung von Hauterkrankungen eingesetzt werden. Andere Einsatzgebiete des Hydroxyectoins und anderer Ectoin-Derivate liegen typischerweise in Gebieten in denen z.B. Trehalose als Zusatzstoff verwendet wird. So können Ectoin-Derivate, wie Hydroxyectoin, als Schutzstoff in getrockneten Hefe- und Bakterienzellen Verwendung finden. Auch pharmazeutische Produkte wie nicht glykosylierte, pharmazeutische wirksame Peptide und Proteine z.B. t-PA können mit Ectoin oder seinen Derivaten geschützt werden.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0

671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Ups, Pflegecremes und Sonnenschutzpräparaten eingesetzt werden.

Dabei wird vorzugsweise eine Pyrimidincarbonsäure gemäß der unten stehenden Formel II eingesetzt,

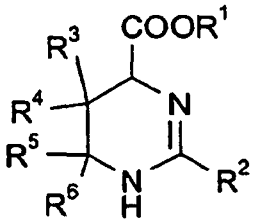

II

worin $R^1$ ein Rest H oder C1-8-Alkyl, $R^2$ ein Rest H oder C1-4-Alkyl und $R^3$, $R^4$, $R^5$ sowie $R^6$ jeweils unabhängig voneinander ein Rest aus der Gruppe H, OH, $NH_2$ und C1-4-Alkyl sind. Bevorzugt werden Pyrimidincarbonsäuren eingesetzt, bei denen $R^2$ eine Methyl- oder eine Ethylgruppe ist und $R^1$ bzw. $R^5$ und $R^6$ H sind. Insbesondere bevorzugt werden die Pyrimidincarbonsäuren Ectoin ((S)-1,4,5,6-Tetrahydro-2-methyl-4-pyrimidin-carbon-säure) und Hydroxyectoin ((S, S)-1,4,5,6-Tetrahydro-5-hydroxy-2-methyl-4-pyrimidin-carbonsäure) eingesetzt. Dabei enthalten die erfindungsgemäßen Zubereitungen derartige Pyrimidincarbonsäuren vorzugsweise in Mengen bis zu 15 Gew.-%. Vorzugsweise werden die Pyrimidincarbonsäuren dabei in Verhältnissen von 100:1 bis 1:100 zu den Verbindungen der Formel Ia eingesetzt, wobei Verhältnisse im Bereich 1:10 bis 10:1 besonders bevorzugt sind.

Unter den Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE-A-41 16 123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist bekannt, dass derartige Zubereitungen z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Erfindungsgemäße Zubereitungen, die neben der Verbindung der Formel Ia zusätzlich eine Aryloxim, vorzugsweise 2-Hydroxy-5-methyllaurophenonoxim enthalten, zeigen überraschende antiinflammatorische Eignung. Dabei enthalten die Zubereitungen vorzugsweise 0,01 bis 10 Gew.-% des Aryloxims, wobei es insbesondere bevorzugt ist, wenn die Zubereitung 0,05 bis 5 Gel-% Aryloxim enthält.

Alle Verbindungen oder Komponenten, die in den Zubereitungen verwendet werden können, sind entweder bekannt und käuflich erwerbbar oder können nach bekannten Verfahren synthetisiert werden. Die Herstellung der Verbindungen nach Formel I wird weiter unten beschrieben.

Die Verbindung der Formel Ia kann in der üblichen Weise in kosmetische oder dermatologische Zubereitungen eingearbeitet werden. Geeignet sind Zubereitungen für eine äußerliche Anwendung, beispielsweise als Creme, Lotion, Gel, oder als Lösung, die auf die Haut aufgesprüht werden kann. Für eine innerliche Anwendung sind Darreichungsformeln wie Kapseln, Dragees, Pulver, Tabletten-Lösungen oder Lösungen geeignet.

Als Anwendungsform der erfindungsgemäßen Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Zubereitung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische

dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Zu den bevorzugten erfindungsgemäßen Zubereitungsformen gehören insbesondere Emulsionen.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:

- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fett-lkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexyl-isostearat, Octyldodecanol, Isotridecylisononanoat, Isoeicosan, 2-Ethylhexylcocoat, $C_{12}$-$_{15}$-Alkylbenzoat, Capryl-Caprinsäure-triglycerid, Dicaprylether.

Besonders vorteilhaft sind Mischungen aus $C_{12}$-$_{15}$-Alkylbenzoat und 2-Ethylhexylisostearat, Mischungen aus $C_{12}$-$_{15}$-Alkylbenzoat und Isotridecylisononanoat sowie Mischungen aus $C_{12}$-$_{15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann auch die Ölphase ferner einen Gehalt an cyclischen oder linearan Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemisch der vorstehend genannten Lösemittel verwendet. Bei alkoholischen Lösemitteln kann Wasser ein weiterer Bestandteil sein.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Formuierung verwendet wird.

In einer bevorzugten Ausführungsform enthalten die erfindungsgemäßen Zubereitungen hydrophile Tenside.

Die hydrophilen Tenside werden bevorzugt gewählt aus der Gruppe der Alkylglucoside, der Acyllactylate, der Betaine sowie der Cocoamphoacetate.

Die Alkylglucoside werden ihrerseits vorteilhaft gewählt aus der Gruppe der Alkylglucoside, welche sich durch die Strukturformel

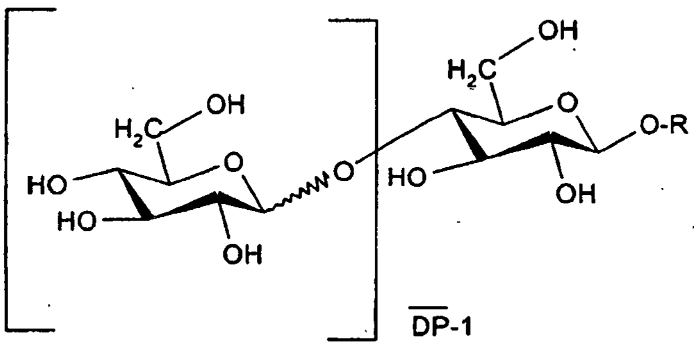

auszeichnen, wobei R einen verzweigten oder unverzweigten Alkylrest mit 4 bis 24 Kohlenstoffatomen darstellt und wobei $\overline{DP}$ einen mittleren Glucosylierungsgrad von bis zu 2 bedeutet.

Der Wert $\overline{DP}$ repräsentiert den Glucösidierungsgrad der erfindungsgemäß verwendeten Alkylglucoside und ist definiert als

$$\overline{DP} = \frac{p_1}{100} \cdot 1 + \frac{p_2}{100} \cdot 2 + \frac{p_3}{100} \cdot 3 + \ldots = \Sigma \frac{p_i}{100} \cdot i$$

Dabei stellen $p_1$, $p_2$, $p_3$ ... bzw. $p_i$ den Anteil der einfach, zweifach dreifach ... i-fach glucosylierten Produkte in Gewichtsprozenten dar. Erfindungsemäß vorteilhaft werden Produkte mit Glucosylierungsgraden von 1-2, insbesondere vorteilhaft von 1, 1 bis 1,5, ganz besonders vorteilhaft von 1,2-1,4, insbesondere von 1,3 gewählt.

Der Wert DP trägt den Umstand Rechnung, dass Alkylglucoside herstellungsedingt in der Regel Gemische aus Mono- und Oligoglucosiden darstellen. Erfindungsgemäß vorteilhaft ist ein relativ hoher Gehalt an Monoglucosiden, typischerweise in der Größenordnung von 40-70 Gew.-%.

Erfindungsgemäß besonders vorteilhaft verwendete Alkylglylcoside werden gewählt aus der Gruppe Octylglucopyranosid, Nonylglucopyranosid, Decylglucopyranosid, Undecylglucopyranosid, Dodecylglucopyranosid, Tetradecylglucopyranosid und Hexadecylglucopyranosid.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren® 1200 (Henkel KGaA), Oramix® NS 10 (Seppic).

Die Acyllactylate werden ihrerseits vorteilhaft gewählt aus der Gruppe der Substanzen, welche sich durch die Strukturformel

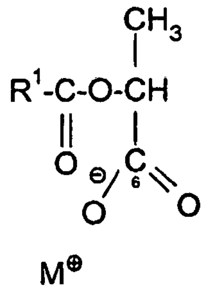

auszeichnen, wobei $R^1$ einen verzweigten oder unverzweigten Alkylrest mit 1 bis 30 Kohlenstoffatomen bedeutet und $M^+$ aus der Gruppe der Alkaliionen sowie der Gruppe der mit einer oder mehreren Alkyl- und/oder mit einer oder mehreren Hydroxyalkylresten substituierten Ammoniumionen gewählt wird bzw. dem halben Äquivalent eines Erdalkalions entspricht.

Vorteilhaft ist beispielsweise Natriumisostearyllactylat, beispielsweise das Produkt Pathionic® ISL von der Gesellschaft American Ingredients Company.

Die Betaine werden vorteilhaft gewählt aus der Gruppe der Substanzen, welche sich durch die Strukturformel

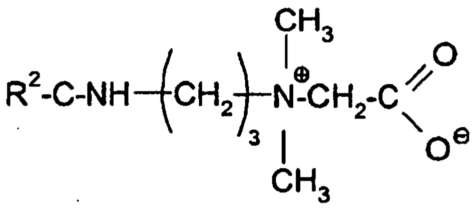

auszeichnen, wobei $R^2$ einen verzweigten oder unverzeigten Alkylrest mit 1 bis 30 Kohlenstoffatomen bedeutet.

Insbesondere vorteilhaft bedeutet $R^2$ einen verzweigten oder unverzweigten Alkylrest mit 6 bis 12 Kohlenstoffatomen.

Vorteilhaft ist beispielsweise Capramidopropylbetain, beispielsweise das Produkt Tego® Betain 810 von der Gesellschaft Th. Goldschmidt AG.

Als erfindungsgemäß vorteilhaftes Cocoamphoacetat wird beispielsweise Natriumcocoamphoacetat gewählt, wie es unter der Bezeichnung Miranol® Ultra C32 von der Gesellschaft Miranol Chemical Corp. erhältlich ist.

Die erfindungsgemäßen Zubereitungen sind vorteilhaft dadurch gekennzeichnet, dass das oder die hydrophilen Tenside in Konzentrationen von 0,01-20 Gew.-% bevorzugt 0,05-10 Gew.-%, besonders bevorzugt 0,1-5 Gew.-%, jeweils bezogen auf das Gesamtgewicht der Zusammensetzung, vorliegt oder vorliegen.

Zu Anwendung werden die erfindungsgemäßen kosmetischen und dermatologischen Zubereitungen in der für Kosmetika üblichen Weise aufdie Haut und/oder die Haare in ausreichender Menge aufgebracht.

Erfindungsgemäße kosmetische und dermatologische Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Bevorzugt werden Emulsionen. O/W-Emulsinen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitere übliche Coemulgatoren in den erfindungsgemäßen bevorzugten O/W-Emulsionen zu verwenden.

Erfindungsgemäß vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch

niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen. Insbesondere bevorzugt sind: Polyethylen-glycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)-stearylether (Steareth-17),Polyethylenglycol(18)stearylether (Steareth-18), Polyethylenglycol(19)stearylether (Steareth-19), Polyethylenglycol(20)-stearylether (Steareth-20), Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)-isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol(20)isostearylether (Isosteareth-20), Polyethylenglycol(13)cetylether (Ceteth-13), Polyethylenglycol(14)cetylether (Ceteth-14), Polyethylené-glycol(15)cetylether (Ceteth-15), Polyethylenglycol(16)cetylether (Ceteth-16), Polyethylenglycol(17)cetylether (Ceteth-17), Polyethylenglycol(18)-cetylether (Ceteth-18), Polyethylenglycol(19)cetylether (Ceteth-19), Polyethylen-glycol(20)cetylether (Ceteth-20), Polyethylen-glycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)-isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20), Polyethylenglycol(12)oleylether (Oleth-12), Polyethylenglycol(13)oleylether (Oleth-13), Polyethylen-glycol(14)oleylether (Oleth-14), Polyethylengfycol(15)oleylether (Oleth-15), Polyethylenglycol(12)laurylether (Laureth-12), Polyethylenglycol(12)-isolaurylether (Isolaureth-12), Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)-cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20).

Es ist ferner von Vorteil, die Fettsäureethoxylate ausfolgender Gruppe zu wählen:

Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethytenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethytenglycol(19)isostearat,
Polyethylenglycol(20)isostearat, Polyethylenglycol(21 )isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,
Polyethylenglycol(16)oleat, Polyethylenglycol(17)oleat,
Polyethylenglycol(18)oleat, Polyethylenglycol(19)oleat,
Polyethylenglycol(20)oleat,

Als ethoxylierte Alkylethercarbonsäure bzw. deren Salz kann vorteilhaft das Natriumlaureth-11-carboxylät verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/cprinat, Polyethylenglycol-(20)glyceryloleat, Polyethylenglycol(20)glycerylisostearat, Polyethylenglycol(18)glyceryloleat(cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als fakultative, dennoch erfindungsgemäß gegebenenfalls vorteilhafte W/O-Emulgatoren können eingesetzt werden:

Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome, Diglycerinester gesättigter und/oder ungesättigter, verzweigter und/oder unverzweigter Alkancarbonsäuren einer Kettenlänge von 8 bis 24, insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkhole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Erfindungsgemäß bevorzugte Zubereitungen eignen sich besonders zum Schutz menschlicher Haut gegen Alterungsprozesse sowie vor oxidativem Stress, d.h. gegen Schädigungen durch Radikale, wie sie z.B. durch Sonneneinstrahlung, Wärme oder andere Einflüsse erzeugt werden. Dabei liegt sie in verschiedenen, für diese Anwendung üblicherweise verwendeten Darreichungsformen vor. So kann sie insbesondere als Lotion oder Emulsion, wie als Creme oder Milch (O/W, W/O. O/W/O, W/O/W), in Form ölig-alkoholischer, ölig-wässriger oder wässrigalkoholischer Gele bzw. Lösungen, als feste Stifte vorliegen oder als Aerosol konfektioniert sein.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und außer der oder den Verbindungen der Formel Ia beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die erfindungsgemäße Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpem.

Ist eine Zubereitung als Aerosol konfektioniert, verwendet man in der Regel die üblichen Treibmittel, wie Alkane, Fluoralkane und Chlorfluoralkane.

Die kosmetische Zubereitung kann auch zum Schutz der Haare gegen fotochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Zubereitung vor oder nach dem Shamponieren, vor oder nach dem Färben oder Entfärben bzw. vor oder nach der Dauerwelle aufgetragen wird. Es kann auch eine Zubereitung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die Zubereitung mit Lichtschutzeigenschaften kann außer der oder den Verbindungen der Formel Ia verschiedene, in diesem Mitteltyp verwendete Adjuvantien enthalten, wie Grenzflächen aktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Weitere Gegenstände der vorliegenden Erfindung sind ein Verfahren zur Herstellung einer Zubereitung, welches dadurch gekennzeichnet ist, dass mindestens eine Verbindung der Formel Ia mit Resten wie oben beschrieben mit einem kosmetisch der dermatologisch oder für nahrungsmittel geeigneten Träger vermischt wird, und die Verwendung

einer Verbindung der Formel Ia zur Herstellung einer Zubereitung.

Die erfindungsgemäßen Zubereitungen können dabei mit Hilfe von Techniken hergestellt werden, die dem Fachmann wohl bekannt sind.

Das Vermischen kann ein Lösen, Emulgieren oder Dispergieren der Verbindung gemäß Formel Ia in dem Träger zur Folge haben.

In einem möglichen Verfahren wird die Verbindung nach Formel I hergestellt durch die Cyclisierung eines entsprechend substituierten o-Hydroxyacetophenones mit einem Anhydrid oder mit einem Acylchlorid unter basischen Bedingungen. Anschließend können die Acyl-Schutzgruppen entfernzt werden. Dabei kann die Umsetzung analog zu Kelly, T ; Kim M.H.; J. Org. Chem. 1992, 57, 1593-97 erfolgen. Alternativ werden die freien Hydroxy-Gruppen acyliert, anschließend folgt folgt eine Baker-Venkatamaran-Umlagerung in basischen Bedingungen mit anschließend Ringschluss unter sauren Bedingungen. Entsprechende Umsetzungen, deren Adaption an die hier gewünschten Verbindungen dem Fachmann keinerlei Probleme bereitet, sind aus der Patentanmeldung WO 2002/060889 bekannt.

Durch übliche Umsetzungen an dem Ringsystem oder Derivatisierung der funktionellen Gruppen können weitere Derivate gemäß Formel I erhalten werden. Die dazu notwendigen Reaktionsbedingung für solche Reaktionen, wie beispielsweise Oxidationen, Reduktionen, Umesterungen, Veretherungen, findet ein Fachmann für derartige Synthesen problemlos in der allgemein zugänglichen Literatur zu organischen Reaktionen.

Es wurde auch festgestellt, dass Verbindungen der Formel Ia stabilisierend auf die Zubereitung wirken können. Bei der Verwendung in entsprechenden Produkten bleiben diese daher auch länger stabil und verändern ihr Aussehen nicht. Insbesondere bleibt auch bei längerdauernder Anwendung bzw. längerer Lagerung die Wirksamkeit der Inhaltsstoffe, z.B. Vitamine, erhalten. Dies ist unter anderem besonders vorteilhaft bei Zusammensetzungen zum Schutz der Haut gegen die Einwirkung von UV-Strahlen, da diese Kosmetika besonders hohen Belastungen durch die UV-Strahlung ausgesetzt sind.

Die positiven Wirkungen von Verbindungen der Formel Ia ergeben deren besondere Eignung zur Verwendung in kosmetischen oder pharmazeutischen Zubereitungen.

Ebenso positiv sind die Eigenschaften von Verbindungen mit der Formel Ia zu werten für eine Verwendung in Nahrungsmitteln oder als Nahrungsergänzungsmittel oder als "functional food". Die weiteren zu Nahrungsmitteln ausgeführten Erläuterungen gelten sinngemäß auch für Nahrungsergänzungsmittel und für "functional food".

Die Nahrungsmittel, die nach der vorliegenden Erfindung mit Verbindungen der Formel Ia angereichert werden können, umfassen alle Materialien, die für den Verzehr durch Tiere oder für den Verzehr durch Menschen geeignet sind, beispielsweise Vitamine und Provitamine davon, Fette, Mineralien oder Aminosäuren ". (Die Nahrungsmittel können fest sein aber auch flüssig, also als Getränk vorliegen). Weitere Gegenstände der vorliegenden Erfindung sind dementsprechend die Verwendung einer Verbindung nach Formel Ia Nahrungsmittelzusatz für die human- oder Tierernährung sowie Zubereitungen, die Nahrungsmittel oder Nahrungsergänzungsmittel sind und entsprechende Träger enthalten.

Nahrungsmittel, die nach der vorliegenden Erfindung mit Verbindungen der Formel Ia angereichert werden können, sind beispielsweise auch Nahrungsmittel, die aus einer einzigen natürlichen Quelle stammen, wie z.B. Zucker, ungesüßter Saft, Nektar oder Püree von einer einzigen Pflanzenspezies, wie z.B. ungesüßter Apfelsaft (z.B. auch eine Mischung verschiedener Sorten Apfelsaft), Grapefruitsaft, Orangensaft, Apfelkompott, Aprikosennektar, Tomatensaft, Tomatensoße, Tomatenpüree usw. Weitere Beispiele für Nahrungsmittel, die nach der vorliegenden Erfindung mit Verbindungen der Formel Ia angereichert werden können, sind Korn oder Getreide einer einzigen Pflanzenspezies und Materialien, die aus derartigen Pflanzenspezies hergestellt werden, wie z.B. Getreidesirup, Roggenmehl, Weizenmehl oder Haferkleie. Auch Mischungen von derartigen Nahrungsmitteln sind geeignet, um nach der vorliegenden Erfindung mit Verbindungen der Formel Ia angereichert zu werden, beispielsweise Multivitaminpräparate, Mineralstoffmischungen oder gezuckerter Saft. Als weitere Beispiele für Nahrungsmittel, die nach der vorliegenden Erfindung mit Verbindungen der Formel Ia angereichert werden können, seien Nahrungsmittelzubereitungen, beispielsweise zubereitete Cerealien, Gebäck, Mischgetränke, speziell für Kinder zubereitete Nahrungsmittel, wie Joghurt, Diätnahrungsmittel, kalorienarme Nahrungsmittel oder Tierfutter, genannt.

Die Nahrungsmittel, die nach der vorliegenden Erfindung mit Verbindungen der Formel Ia angereichert werden können, umfassen somit alle genießbaren Kombinationen von Kohlehydraten, Lipiden, Proteinen, anorganischen Elementen, Spurenelementen, Vitaminen, Wasser oder aktiven Metaboliten von Pflanzen und Tieren.

Die Nahrungsmittel, die nach der vorliegenden Erfindung mit Verbindungen der Formel Ia angereichert werden können, werden vorzugsweise oral angewendet, z.B. in Form von Speisen, Pillen, Tabletten, Kapseln, Pulver, Sirup, Lösungen oder Suspensionen.

Die mit Verbindungen der Formel Ia angereicherten erfindungsgemäßen Nahrungsmittel können mit Hilfe von Techniken hergestellt werden, die dem Fachmann wohl bekannt sind.

Durch ihre Wirkung eignen sich Verbindungen der Formel Ia auch als Arzneimittelinhaltsstoff. Verbindungen der Formel Ia können beispielsweise zur vorbeugenden Behandlungen von Entzündungen und Allergien der Haut sowie in bestimmten Fällen zur Verhütung bestimmter Krebsarten verwendet werden. Insbesondere eignen sich Verbindungen der Formel Ia zur Herstellung eines Arzneimittels zur Behandlung von Entzündungen, Allergien und Irritationen, insbe-

sondere der Haut. Ferner können Arzneimittel hergestellt werden in einer Wirkung als Venentonikum, als Hemmstoff für Cuperose, als Hemmstoff chemischer, physikalischer oder aktinischer Erytheme, als Mittel zur Behandlung empfindlicher Haut, als Dekongestionsmittel, als Entwässerungsmittel, als Mittel zum Schlankmachen, als Antifaltenmittel, als Stimulatoren der Synthese von Komponenten der extrazellulären Matrix, als stärkendes Mittel zur Verbesserung der Hautelastizität und als Antialterungsmittel. Weiter zeigen in diesem Zusammenhang bevorzugte Verbindungen der Formel Ia antiallergische und antiinflammatorische und antiirritative Wirkungen. Sie eignen sich daher zur Herstellung von Arzneimitteln zur Behandlung von Entzündungen oder allergischen Reaktionen.

Im folgenden wird die Erfindung anhand von Beispielen näher erläutert.

**Beispiel 1: Darstellung von 5,7-Diacetoxy-3-acetyl-2-methyl-chromen-4-one**

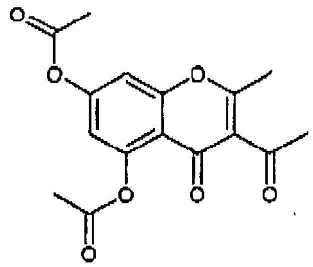

2,4,6-Trihydroxyacetophenon wird in Essigsäureanhydrid gelöst vorgelegt und Natriumacetat zugegeben. Die Suspension wird 10h unter Rückfluss rühren gelassen. Anschließend wird die Reaktionsmischung in ca. 300ml Eiswasser gegossen und 2x mit Essigsäureethylester (EEE) extrahiert, die org. Phasen werden vereinigt, 3x mit $H_2O$-VE gewaschen. Die verbleibende Lösung wird weiter mit $Na_2HCO_3$ -Lsg. gewaschen. Die organische Phase wird über $Na_2SO_4$ getrocknet, filtriert und einrotiert.

$^1$H NMR (300 MHz) in DMSO $\delta$ (ppm) : 7.1 (d, 1H), 7.4 (d, 1H)

MS (m/z) : 318 ($M^+$)

**Beispiel 2: Darstellung von 5,7-Dihydroxy-2-methyl-chromen-4-on**

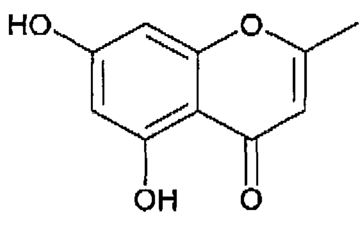

5,7-Diacetoxy-3-acetyl-2-methyl-chromen-4-on wird mit 40ml 10%iger Natriumcarbonatlösung 1 h unter Rückfluss gekocht. Nach dem Abkühlen wird die Suspension mit 2N HCl auf pH ca. 6 eingestellt und gekühlt. Der Niederschlag wird abfiltriert. Es werden 0,6 g sehr helles braunes Pulver erhelten. ($T_M$ = 279,9°C)

$^1$H NMR (300 MHz) in DMSO $\delta$ (ppm): 2.3 (s, 3H), 6.15 (s, 1H), 6.18 (d, 1H), 6.3 (d, 1 H), 10.8 (bs, 1O<u>H</u>). 12,8 (s, 1O<u>H</u>)

MS (m/z) : 192 ($M^+$)

**Beispiel 3: Wirksamkeitsuntersuchungen**

**Beispiel 3: Antiinflammatorische Eigenschaften (PGE2 assay)**

Menschliche Keratinozyten der Zelliinie NCTC R13 werden bei 37°C in einer 5%igen CO2 Atmosphere mit dem Kulturmedium DMEM (Life Technologies) 24 h vorkultiviert. Die Zellen werden 24 Stunden mit LU behandeln, dann wird das Kulturmedium entfernt. Ein neues Kulturmedium mit dem Inflammations-auslösende Wirkstoff Phorbol Myristat Acetat (PMA; 0,1 g/ml) und LU wird zugegeben. Nach 24 Stunden Inkubation wird die Kulturmitte gesammelt und analysiert. Mittels eines ELISA-Kit DE0100 (R&D Systems) wird die Freisetzung benutzt um der Freigabe des Inflammations-Markers Prostaglandin E2 (PGE2) untersucht. (Tabelle).

Tabelle: PGE2-Freisetzung von Humankeratinozyten

| | 0,2 mM LU | 0,04 MM LEU | 0,008 mM LU | Kontrolle (nur PMA) | neg. Kontrolle (ohne PMA) |
|---|---|---|---|---|---|
| PGE2 (ng/ml) | 6,31 | 226,39 | 340,62 | 302,02 | 0,018 |
| Anzahl der Tests | 3 | 3 | 3 | 3 | 3 |
| Standardabweichung | 0,5 | 11,92 | 17,93 | 9,31 | 0,001 |
| % zur Kontrolle | 2 | 75 | 113 | 100 | 0 |

5,7-Dihydroxy-2-methyl-chromen-4-on zeigt in der Dosierung 0,2 mM und 0,04 mM eine deutliche Reduktion der PGE2-Freisetzung unter PMA-Einwirkung.

**Beispiel 3b: Wirkung auf die Aktivität der Leukozyten Elastase**

5,7-Dihydroxy-2-methyl-chromen-4-on in Tris-Puffer (500mM) wird mit Elastase (aus Human-Leukozyten; Sigma E8140; 100mU/well) 10 Minuten auf Eis inkubiert. Anschließend werden 5 μg/well Elastin zugegeben und die Platten 2 h bei 37°C inkubiert. Die Flureszenz wurde mit einem Spectromax Gemini Spektrometer (Molecular Devices) bei λex = 485 nm und λem = 538 nm bestimmt.

Tabelle: Leukozyten Elastase Aktivität

| | 200 μM LU | 40 μM LU | Kontrolle (0,1 mM AAPV) | neg. Kontrolle |
|---|---|---|---|---|
| Fluoreszenzintensität | 1096,4 | 2195,9 | 96,0 | 2430,2 |
| Anzahl der Tests | 3 | 3 | 3 | 3 |
| Standardabweichung | 53 | 33 | 7 | 62 |
| % zur Kontrolle | 45 | 90 | 4 | 100 |
| % quenching | 19 | 0 | 0 | - |
| Inhibierung (%) | 45 | 10 | 96 | - |

5,7-Dihydroxy-2-methyl-chromen-4-on zeigt in der Dosierung 200 μM und 40 μM eine deutliche Inhibierung der Elastase - Aktivität.

**Beispiel 3c: Wirkung auf die Aktivität der Hyaluronidase**

5,7-Dihydroxy-2-methyl-chromen-4-on in Phosphat-Puffer (0,1 M) wird mit Hyaluronidase (HYAL, Sigma type IV-S, H3884; 1 mg/l in Phosphat-Puffer (0,1 M)) vorinkubiert. Anschließend wird Hyaluronsäure (AH, Sigma H-1876; 1,2 mg/ml) zugegeben und die Mischung 1 h bei 37°C inkubiert. Anschließend wird die verbleibende Hyaluronsäure (HA) mit Serum Albumin (BSA, Sigma A7888) gefällt und photometrisch bestimmt.

Tabelle: Hyaluronidase Aktivität

| | 25 mM LU | 12,5 mM LU | 6,25 mM LU | 3,12 5 mM LU | kontrolle (nur HA, ohne Enzym) | Kontrolle (ohne LU) |
|---|---|---|---|---|---|---|
| Hyaluronidase Aktivität | 37 | 63 | 81 | 91 | 0 | 100 |
| Hyaluronidase Inhibierung | 63 | 37 | 19 | 9 | - | 0 |

5,7-Dihydroxy-2-methyl-chromen-4-on zeigt in den untersuchten Dosierungen eine deutliche Inhibierung der Hyaluronidase - Aktivität. 50% Hemmung ($IC_{50}$) wird bei etwa 20 mM 6,7-Dihydroxy-2-methyl-ohromen-4-on erreicht.

**Beispiel 4: Zubereitungen**

Im folgenden werden beispielhaft Rezepturen für kosmetische Zubereitungen angegeben, die Verbindungen nach Beispiel 2 enthalten. Im übrigen sind die INCI-Bezeichnungen der handelsüblichen Verbindungen angegeben.

UV-Pearl , OMC steht für die Zubereitung mit der INCI-Bezeichnung: Water (for EU: Aqua), Ethylhexyl Methoxycinnamate, Silica, PVP, Chlorphenesin, BHT; diese Zubereitung ist im handel unter der Bezeichnung Eusolex®UV Pearl™OMC von der Merck KGaA, Darmstadt erhältlich.

Die anderen in den Tabellen angegebenen UV-Pearl sind jeweils analog zusammengesetzt, wobei OMC gegen die angegebenen UV-Filter ausgetauscht wurde.

Tabelle 1 W/O-Emulsionen (Zahlen in Gew.-%)

| | 1-1 | 1-2 | 1-3 | 1-4 | 1-5 | 1-6 | 1-7 | | | | |
|---|---|---|---|---|---|---|---|---|---|---|---|
| Titanium dioxide | | 2 | 5 | | | | 3 | | | | |
| 2-Methyl-5,7-dihydroxy-chromen-4-on | 5 | 3 | 2 | 1 | 2 | 1 | 1 | | | | |
| Zinc oxide | | | | | | 2 | | | | | |
| UV-Pearl, OMC | 30 | 15 | 15 | 15 | 15 | 15 | 15 | | | | |
| Polyglyceryl-3-Dimerate | 3 | 3 | 3 | 3 | 3 | 3 | 3 | | | | |
| Cera Alba | 0,3 | 0,3 | 0,3 | 0,3 | 0,3 | 0,3 | 0,3 | | | | |
| Hydrogenated Castor Oil | 0,2 | 0,2 | 0,2 | 0,2 | 0,2 | 0,2 | 0,2 | | | | |
| Paraffinium Liquidum | 7 | 7 | 7 | 7 | 7 | 7 | 7 | | | | |
| Caprylic/Capric Triglyceride | 7 | 7 | 7 | 7 | 7 | 7 | 7 | | | | |
| Hexyl Laurate | 4 | 4 | 4 | 4 | 4 | 4 | 4 | | | | |
| PVP/Eicosene Copolymer | 2 | 2 | 2 | 2 | 2 | 2 | 2 | | | | |
| Propylene Glycol | 4 | 4 | 4 | 4 | 4 | 4 | 4 | | | | |
| Magnesium Sulfate | 0,6 | 0,6 | 0,6 | 0,6 | 0,6 | 0,6 | 0,6 | | | | |
| Tocopherol | 0,5 | 0,5 | 0,5 | 0,5 | 0,5 | 0,5 | 0,5 | | | | |
| Tocopheryl Acetate | 0,5 | 0,5 | 0,5 | 0,5 | 0,5 | 0,5 | 0,5 | | | | |
| Cyclomethicone | 0,5 | 0,5 | 0,5 | 0,5 | 0,5 | 0,5 | 0,5 | | | | |
| Propylparabene | 0,05 | 0,05 | 0,05 | 0,05 | 0,05 | 0,05 | 0,05 | | | | |
| Methylparabene | 0,15 | 0,15 | 0,15 | 0.15 | 0,15 | 0,15 | 0,15 | | | | |
| Water | ad 100 | ad 100 | ad 100 | ad 100 | ad 100 | ad 100 | ad 100 | | | | |
| | 1-8 | 1-9 | 1-10 | 1-11 | 1-12 | 1-13 | 1-15 | 1-16 | 1-16 | 1-17 | 1-18 |
| Titanium dioxide | | 2 | 5 | | | | | | | 3 | 3 |
| Benzylidene malonate polysiloxane | | | | 1 | | | | | 1 | 1 | |
| Zinc oxide | | | | | | | | 5 | 2 | | |
| 2-Methyl-5,7-dihydroxy-chromen-4-on | 5 | 5 | 5 | 5 | 7 | 5 | 5 | 5 | 5 | 5 | 8 |
| UV-Pearl , OCR | | 10 | | | | | | | | | 5 |

(fortgesetzt)

| | 1-8 | 1-9 | 1-10 | 1-11 | 1-12 | 1-13 | 1-15 | 1-16 | 1-16 | 1-17 | 1-18 |
|---|---|---|---|---|---|---|---|---|---|---|---|
| UV-Pearl, EthylhexylDimethy[PABA | | | 10 | | | | | | | | |
| UV-Pearl, Homosalate | | | | 10 | | | | | | | |
| UV-Pearl, Ethylhexyl salicylate | | | | | 10 | | | | | | |
| UV-Pearl , OMC, BP-3 | | | | | | 10 | | | | | |
| UV-Pearl, OCR, BP-3 | | | | | | | 10 | | | | |
| UV-Pearl, Ethylhexyl Dimethyl PABA, BP-3 | | | | | | | | 10 | | | |
| UV-Pearl, Homosalate, BP-3 | | | | | | | | | 10 | | |
| UV-Pearl, Ethylhexyl salicylate, BP-3 | | | | | | | | | | 10 | |
| BMDBM | | | | | | | | | | | 2 |
| UV-Pearl OMC, 4-Methylbenzylidene Camphor | 25 | | | | | | | | | | |
| Polyglyceryl-3-Dimerate | 3 | 3 | 3 | 3 | 3 | 3 | 3 | 3 | 3 | 3 | 3 |
| Cera Alba | 0,3 | 0,3 | 0,3 | 0,3 | 0,3 | 0,3 | 0,3 | 0,3 | 0,3 | 0,3 | 0,3 |
| Hydrogenated Castor Oil | 0,2 | 0,2 | 0,2 | 0,2 | 0,2 | 0,2 | 0,2 | 0,2 | 0.2 | 0,2 | 0,2 |
| Paraffinium Liquidum | 7 | 7 | 7 | 7 | 7 | 7 | 7 | 7 | 7 | 7 | 7 |
| Caprylic/Capric Triglyceride | 7 | 7 | 7 | 7 | 7 | 7 | 7 | 7 | 7 | 7 | 7 |
| Hexyl Laurate | 4 | 4 | 4 | 4 | 4 | 4 | 4 | 4 | 4 | 4 | 4 |
| PVP/Eicosene Copolymer | 2 | 2 | 2 | 2 | 2 | 2 | 2 | 2 | 2 | 2 | 2 |
| Propylene Glycol | 4 | 4 | 4 | 4 | 4 | 4 | 4 | 4 | 4 | 4 | 4 |
| Magnesium Sulfate | 0,6 | 0,6 | 0,6 | 0,6 | 0,6 | 0,6 | 0,6 | 0,6 | 0,6 | 0,6 | 0,6 |
| Tocopherol | 0,5 | 0,5 | 0,5 | 0,5 | 0,5 | 0,5 | 0,5 | 0,5 | 0,5 | 0,5 | 0,5 |
| Tocopheryl Acetate | 0,5 | 0,5 | 0,5 | 0,5 | 0,5 | 0,5 | 0,5 | 0,5 | 0,5 | 0,5 | 0,5 |
| Cyclomethicone | 0,5 | 0,5 | 0.5 | 0,5 | 0,5 | 0,5 | 0,5 | 0,5 | 0,5 | 0,5 | 0,5 |
| Propylparabene | 0,05 | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 | 0,15 |
| Water | ad 100 | | | | | | | | | | |

Tabelle 2 : O/W - Emulsionen, Zahlen in Gew.-%

| | 2-1 | 2-2 | 2-3 | 2-4 | 2-5 | 2-6 | 2-7 | 2-8 | 2-9 | 2-10 |
|---|---|---|---|---|---|---|---|---|---|---|
| Titanium dioxide | | | | | | | 3 | 3 | | 2 |
| Benzylidene malonate polysiloxane | 1 | 2 | | | | 1 | 1 | | 1 | 0,5 |
| 7,8,3',4'-Tetrahydroxyflavon | | | | 1 | 2 | | | | 1 | 1 |
| Ethyl 5,7-dihydroxy-chromen-4-on-2-carboxylat | 1 | 3 | | 2 | | 5 | | 5 | 2 | |
| 2-Methyl-5,7-dihydroxy-chromen-4-on | 5 | 5 | 5 | 5 | 5 | 5 | 5 | 5 | 5 | 5 |
| Methylene Bis-Benztriazolyl Tetramethylbutylphenol | | | 1 | 2 | 1 | | | 1 | 1 | 0,5 |
| Zinc oxide | | | | | 5 | 2 | | | | 2 |
| UV-Pearl, OMC | 15 | 15 | 15 | 15 | 15 | 15 | 15 | 15 | 15 | 15 |
| Caprylic/Capric Triglyceride | 14 | 14 | 14 | 14 | 14 | 14 | 14 | 14 | 14 | 14 |
| Oleyl Oleate | | | | | | | | | | |
| Propylene Glycol | | | | | | | | | | |
| Glyceryl Stearate SE | 6 | 6 | 6 | 6 | 6 | 6 | 6 | 6 | 6 | 6 |
| Stearic Acid | 2 | 2 | 2 | 2 | 2 | 2 | 2 | 2 | 2 | 2 |
| Persea Gratissima | 8 | 8 | 8 | 8 | 8 | 8 | 8 | 8 | 8 | 8 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Glyceryl Stearate, Ceteareth-20, Ceteareth-10, Cetearyl Alcohol, Cetyl Palmitate | | | | | | | | | | |
| Ceteareth-30 | | | | | | | | | | |
| Dicaprylyl Ether | | | | | | | | | | |
| Hexyldecanol, Hexyldexyllaurate | | | | | | | | | | |
| Cocoglycerides | | | | | | | | | | |
| Tromethamine | | | | | | | | | | |
| Glycerin | 3 | 3 | 3 | 3 | 3 | 3 | 3 | 3 | 3 | 3 |
| Water | ad 100 | ad 100 | ad 100 | ad 100 | ad 100 | ad 100 | ad 100 | ad 100 | ad 100 | ad 100 |

Tabelle 3: Gele, Zahlen in Gew.-%

| | 3-1 | 3-2 | 3-3 | 3-4 |
|---|---|---|---|---|
| a = aqueaous gel | | | | |
| Titanium dioxide | | | | 3 |
| 2-Methyl-5,7-dihydroxy-chromen-4-on | 1 | 2 | 1 | 1 |
| Ethyl 5,7-dihydroxy-chromen-4-on-2-carboxylat | 2 | | 2 | |
| Benzylidene malonate polysiloxane | 1 | 2 | 1 | 1 |

(fortgesetzt)

| | 3-1 | 3-2 | 3-3 | 3-4 |
|---|---|---|---|---|
| Methylene Bis-Benztriazolyl Tetramethylbutylphenol | | | | |
| Zinc oxide | 2 | | 2 | |
| UV-Pearl , Ethylhexyl Mehtoxycinnamat | 15 | 15 | 15 | 15 |
| 4-Methylbenzyliden Camphor | | 2 | | |
| Butylmethoxydibenzoylmethane | | | | |
| Phenylbenzimidazole Sulfonic Acid | | | | |
| Prunus Dulcis | 5 | 5 | 5 | 5 |
| Tocopheryl Acetate | 0,5 | 0,5 | 0,5 | 0,5 |
| Caprylic/Capric Triglyceride | 3 | 3 | 3 | 3 |
| Octyldodecanol | 2 | 2 | 2 | 2 |
| Decyl Oleate | 2 | 2 | 2 | 2 |
| PEG-8 (and) Tocopherol (and) Ascorbyl Palmitate (and) Ascorbic Acid (and) Citric Acid | 0,05 | 0,05 | 0,05 | 0,05 |
| Sorbitol | 4 | 4 | 4 | 4 |
| Polyacrylamide (and) C13-14 Isoparaffin (and) Laureth-7 | 3 | 3 | 3 | 3 |
| Propylparabene | 0,05 | 0,05 | 0,05 | 0,05 |
| Methylparabene | 0,15 | 0,15 | 0,15 | 0,15 |
| Tromethamine | | | | |
| Water | ad 100 | ad 100 | ad 100 | ad 100 |

**Patentansprüche**

1. Nicht-therapeutische Verwendung der Verbindung der Formel Ia

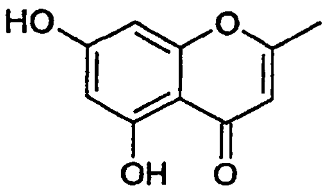

Ia

oder einer Zubereitung enthaltend die Verbindung der Formel Ia zur Prophylaxe gegen oder Reduktion von Hautunebenheiten. 7

2. Nicht-therapeutische Verwendung gemäß Anspruch 1 zur Prophylaxe gegen oder Reduktion von Falten, feinen Linien, rauher Haut oder großporiger Haut.

3. Verwendung der Verbindung der Formel Ia gemäß Anspruch 1 zur Herstellung einer Zubereitung geeignet zur Prophylaxe und/oder Behandlung von Hautkrankheiten, die mit einer Störung der Keratinisierung verbunden sind, die Differenzierung und Zellproliferation betrifft, zur Behandlung anderer Störungen der Keratinisierung, zur Behandlung anderer Hauterkrankungen, die mit einer Störung der Keratinisierung zusammenhängen und eine entzündliche und/oder immunoallergische Komponente haben und des psoriatischen Rheumas und der Haut-atopien oder der respiratorischen Atopie oder auch der Hypertrophie des Zahnfleisches.

4. Verwendung gemäß Anspruch 3 zur Behandlung der Akne vulgaris, Akne comedonicä, der polymorphen Akne, der

Akne rosaceae, der nodulären Akne, der Akne conglobata, der alters-bedingten Aknen, der als Nebenwirkung auftretenden Aknen, wie der Akne solaris, der medikamenten-bedingten Akne oder der Akne professionalis, der Ichtyosen, der ichtyosi-formen Zustände, der Darrier-Krankheit, der Keratosis palmoplantaris, der Leukoplasien, der leukoplasiformen Zustände, der Haut- und Schleimhaut-flechten (Buccal) (Lichen) und aller Formen der Psoriasis, die die Haut, die Schleimhäute und die Finger und Zehennägel betreffen.

**5.** Verwendung der Verbindung der Formel Ia gemäß Anspruch 1 zur Beruhigung von empfindlicher und gereizter Haut, zur vorbeugenden Regulation der Kollagen-, Hyaluronsäure-, Elastinsynthese und Regulation der Transkription und Translation matrixabbauender Enzyme.

**6.** Zubereitung enthaltend die Verbindung gemäß Formel Ia gemäß Anspruch 1, sowie mindestens einen weiteren hautpflegenden Inhaltsstoff und mindestens einen für topische Anwendungen geeigneten Träger.

**7.** Zubereitung nach Anspruch 6, **dadurch gekennzeichnet, dass** die Zubereitungen die Verbindung der Formel Ia in einer Menge von 0,01 bis 20 Gew.-% enthält.

**8.** Zubereitung nach mindestens einem der vorhergehenden Ansprüche, **dadurch gekennzeichnet, dass** es sich bei mindestens einem weiteren hautpflegenden Inhaltsstoff um ein oder mehrere Antioxidantien und/oder Vitamine handelt, wobei die Zubereitung kein Retinol-Derivat enthält.

**9.** Zubereitung nach mindestens einem der vorhergehenden Ansprüche, wobei die Zubereitung einen oder mehrere UV-Filter enthält.

**10.** Zubereitung nach Anspruch 9, **dadurch gekennzeichnet, dass** der UV-Filter ausgewählt wird aus der Gruppe 3-(4'-Methylbenzyliden)-di-kampfer, 1-(4-tert-Butylphenyl)-3-(4-methoxyphenyl)propan-1,3-dion, 4-Isopropyldibenzoylmethan, 2-Hydroxy-4-methoxybenzophenon, Methoxyzimtsäureoctylester, 3,3,5-Trimethyl-cyclohexylsalicylat, 4-(Dimethylamino)benzoesäure2-ethylhexylester, 2-Cyano-3,3-di-phenylacrylsäure-2-ethylhexylester, 2-Phenylbenzimidazol-5-sulfonsäure sowie ihre Kalium-, Natrium- und Triethanolaminsalze.

**11.** Zubereitung nach mindestens einem der vorhergehenden Ansprüche, **dadurch gekennzeichnet, dass** es sich bei mindestens einem weiteren hautpflegenden Inhaltsstoff um Pyrimidincarbonsäuren und/oder Aryloxime handelt.

**12.** Verfahren zur Herstellung einer Zubereitung **dadurch gekennzeichnet, dass** die Verbindung der Formel Ia gemäß Anspruch 1 mit einem kosmetisch oder dermatologisch oder für Nahrungsmittel geeignetem Träger vermischt wird.

## Claims

**1.** Non-therapeutic use of the compound of the formula Ia

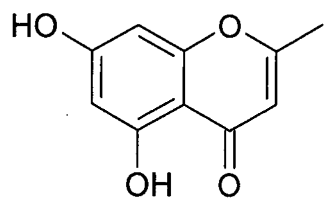

Ia

or a preparation comprising the compound of the formula Ia for prophylaxis against or reduction of skin unevenness.

**2.** Non-therapeutic use according to Claim 1 for prophylaxis against or reduction of wrinkles, fine lines, rough skin or large-pored skin.

**3.** Use of the compound of the formula Ia according to Claim 1 for preparing a preparation which is suitable for prophylaxis and/or treatment of skin diseases which are associated with a keratinisation defect relating to differentiation and cell proliferation, for the treatment of other keratinisation defects, for the treatment of other skin diseases which are associated with a keratinisation defect and have an inflammatory and/or immunoallergic component and of psoriatic rheumatism and skin atopies or respiratory atopy or also hypertrophy of the gums.

**4.** Use according to Claim 3 for the treatment of acne vulgaris, acne comedonica, polymorphic acne, acne rosaceae, nodular acne, acne conglobata, age-related acne, acne occurring as a side effect, such as acne solaris, medicament-induced acne or acne professionalis, ichthyosis, ichthyosiform states, Darier's disease, keratosis palmoplantaris, leukoplasias, leukoplasiform states, skin and mucosal eczema (buccal) (lichen) and all forms of psoriasis relating to the skin, the mucous membranes and finger- and toenails,

**5.** Use of the compound of the formula Ia according to Claim 1 for calming sensitive and irritated skin, for the preventative regulation of collagen, hyaluronic acid and elastin synthesis and regulation of the transcription and translation of matrix-degrading enzymes.

**6.** Preparation comprising the compound of the formula Ia according to Claim 1 and at least one further skin-care ingredient and at least one vehicle which is suitable for topical applications.

**7.** Preparation according to Claim 6, **characterised in that** the preparation comprises the compound of the formula Ia in an amount of 0.01 to 20% by weight.

**8.** Preparation according to at least one of the preceding claims, **characterised in that** the at least one further skin-care ingredient is one or more antioxidants and/or vitamins, where the preparation preferably does not comprise a retinol derivative.

**9.** Preparation according to at least one of the preceding claims, where the preparation comprises one or more UV filters.

**10.** Preparation according to Claim 9, **characterised in that** the UV filter is selected from the group consisting of 3-(4'-methylbenzylidene)-di-camphor, 1-(4-tert-butylphenyl)-3-(4-methoxyphenyl)propane-1,3-dione, 4-isopropyldibenzoylmethane, 2-hydroxy-4-methoxybenzo-phenone, octyl methoxycinnamate, 3,3,5-trimethylcyclohexyl salicylate, 2-ethylhexyl 4-(dimethylamino)benzoate, 2-ethylhexyl 2-cyano-3,3-diphenylacrylate, 2-phenylbenzimidazole-5-sulfonic acid and potassium, sodium and triethanolamine salts thereof.

**11.** Preparation according to at least one of the preceding claims, **characterised in that** the at least one further skin-care ingredient is a pyrimidinecarboxylic acid and/or aryl oxime.

**12.** Process for preparing a preparation, **characterised in that** the compound of the formula Ia according to Claim 1 is mixed with a vehicle which is suitable cosmetically or dermatologically or for food.

## Revendications

**1.** Utilisation non thérapeutique du composé de formule Ia

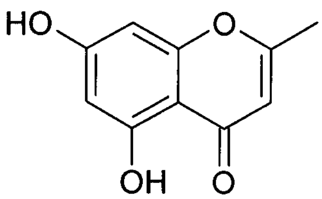

Ia

ou une préparation comprenant le composé de formule Ia pour une prophylaxie contre les irrégularités de la peau ou leur réduction.

**2.** Utilisation non thérapeutique selon la revendication 1, pour la prophylaxie contre les rides, les rides, une peau rêche ou une peau à pores larges, ou leur réduction.

**3.** Utilisation du composé de formule Ia selon la revendication 1, pour l'élaboration d'une préparation qui est convenable pour la prophylaxie et/ou le traitement de maladies de la peau qui sont associées à un défaut de kératinisation lié à une différentiation et une prolifération cellulaire, pour le traitement d'autres défauts de kératinisation, pour le traitement d'autres maladies de la peau qui sont associées à un défaut de kératinisation et possèdent une composante inflammatoire et/ou immuno-allergique et du rhumatisme psoriasique, et des atopies de la peau ou de l'atopie

respiratoire ou également de l'hypertrophie des gencives.

**4.** Utilisation selon la revendication 3, pour le traitement de l'acné vulgaire, de l'acné comédonienne, de l'acné polymorphe, de l'acné rosacée, de l'acné nodulaire, de l'acné conglobata, de l'acné liée à l'âge, de l'acné apparaissant comme effet secondaire, telle que l'acné solaire, l'acné induite par des médicaments ou l'acné professionnelle, l'ichthyose, les états ichthyosiformes, la maladie de Darier,
la kératose palmoplantaire, les leucoplasies, les états leucoplasi-formes, l'eczéma cutané et mucosal (buccal) (lichen) et toutes les formes de psoriasis liées à la peau, aux muqueuses et aux ongles des mains et des pieds,

**5.** Utilisation du composé de formule la selon la revendication 1, pour calmer une peau sensible et irritée, pour la régulation préventive de la synthèse du collagène, de l'acide hyaluronique et de l'élastine et la régulation de la transcription et de la traduction des enzymes de dégradation de la matrice.

**6.** Préparation comprenant le composé de formule la selon la revendication 1 et au moins un autre ingrédient de soin de la peau et au moins un véhicule convenable pour des applications topiques.

**7.** Préparation selon la revendication 6, **caractérisée en ce que** la préparation comprend le composé de formule la selon une quantité allant de 0,01 à 20% en poids.

**8.** Préparation selon au moins l'une des revendications précédentes, **caractérisée en ce que** le au moins un autre ingrédient de soin de la peau est un ou plusieurs parmi des antioxydants et/ou des vitamines, où la préparation ne comprend de préférence pas de dérivé de rétinol.

**9.** Préparation selon au moins l'une des revendications précédentes, **caractérisée en ce que** la préparation comprend un ou plusieurs filtres anti-UV.

**10.** Préparation selon la revendication 9, **caractérisée en ce que** le filtre anti-UV est choisi dans le groupe constitué par le 3-(4'-méthyl-benzylidène)-dl-camphre, la 1-(4-tertio-butylphényl)-3-(4-méthoxy-phényl)propane-1,3-dione, le 4-isopropyldibenzoylméthane, la 2-hydroxy-4-méthoxybenzophénone, le méthoxycinnamate d'octyle, le salicylate de 3,3,5-triméthylcyclohexyle, le 4-(diméthylamino)-benzoate de 2-éthylhexyle, le 2-cyano-3,3-diphénylacrylate de 2-éthylhexyle, l'acide 2-phénylbenzimidazole-5-sulfonique et les sels de potassium, sodium et triéthanolamine de celui-ci.

**11.** Préparation selon au moins l'une des revendications précédentes, **caractérisée en ce que** le au moins un autre ingrédient de soin de la peau est un acide pyrimidinecarboxylique et/ou un aryl oxime.

**12.** Procédé d'élaboration d'une préparation, **caractérisé en ce que** le composé de formule la selon la revendication 1 est mélangé avec un véhicule qui est convenable sur le plan cosmétique ou dermatologique ou alimentaire.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9319743 A
- WO 0202074 A
- WO 9407462 A
- EP 0304802 A
- EP 0424444 A
- US 6019992 A
- EP 1216692 A
- JP 5301813 A
- JP 9188608 A
- JP 10194919 A
- JP 10114640 A
- DE 10244282
- DE 10232595
- US 6242099 B1
- WO 0009652 A
- WO 0072806 A
- WO 0071084 A
- EP 0671161 A
- DE 4116123 A
- DE OS4308282 A
- WO 2002060889 A


### In der Beschreibung aufgeführte Nicht-Patentliteratur


- **K. LEMANSKA ; H. SZYMUSIAK ; B. TYRAKOWSKA ; R. ZIELINSKI ; I.M.C.M. RIETJENS.** *Current Topics in Biophysics,* 2000, vol. 24 (2), 101-108
- **C.A. RICE-EVANS ; N.J. MILLER ; G. PAGANGA.** *Trends in Plant Science,* 1997, vol. 2 (4), 152-159
- **K. LEMANSKA ; H. SZYMUSIAK ; B. TYRAKOWSKA ; R. ZIELINSKI ; A.E.M.F. SOFFERS ; I.M.C.M. RIETJENS.** *Free Radical Biology&Medicine,* 2001, vol. 31 (7), 869-881
- **E. A. GALINSKI et al.** *Eur. J. Biochem.,* 1985, vol. 149, 135-139
- **KELLY, T ; KIM M.H.** *J. Org. Chem.,* 1992, vol. 57, 1593-97